(19) Europäisches Patentamt / European Patent Office / Office européen des brevets

(11) **EP 3 794 971 A1**

(12) **EUROPEAN PATENT APPLICATION**

(43) Date of publication:
    **24.03.2021 Bulletin 2021/12**

(51) Int Cl.:
    **A24F 40/10** (2020.01)     **A24F 40/40** (2020.01)
    **A61M 15/00** (2006.01)

(21) Application number: **19198578.7**

(22) Date of filing: **20.09.2019**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(71) Applicant: **NERUDIA LIMITED
    Liverpool Merseyside L24 9HP (GB)**

(72) Inventor: **The designation of the inventor has not
    yet been filed**

(74) Representative: **Mewburn Ellis LLP
    Aurora Building
    Counterslip
    Bristol BS1 6BX (GB)**

(54) **SMOKING SUBSTITUTE APPARATUS**

(57)     A smoking substitute apparatus. The smoking substitute apparatus comprising: a vaporiser chamber, including a vaporiser configured to vaporise a vaporisable liquid; a primary airflow path, which passes from a first air inlet of the smoking substitute apparatus through the vaporiser chamber, to an outlet of the smoking substitute apparatus; a secondary airflow path, which passes from a second air inlet of the smoking substitute apparatus to the outlet of the smoking substitute apparatus, said secondary airflow path bypassing the vaporiser chamber and passing through one or more bypass air ducts; wherein one or more adjustable airflow restrictors are disposed along either or both of the primary airflow path and the secondary airflow path, said adjustable airflow restrictors being adjustable by a user of the device to vary a draw resistance of the smoking substitute apparatus.

FIG. 21

EP 3 794 971 A1

**Description**

*Field of the Invention*

The present invention relates to a smoking substitute apparatus and, in particular, a smoking substitute apparatus that is able to deliver nicotine to a user in an effective manner.

*Background*

The smoking of tobacco is generally considered to expose a smoker to potentially harmful substances. It is thought that a significant amount of the potentially harmful substances are generated through the burning and/or combustion of the tobacco and the constituents of the burnt tobacco in the tobacco smoke itself.

Low temperature combustion of organic material such as tobacco is known to produce tar and other potentially harmful by-products. There have been proposed various smoking substitute systems in which the conventional smoking of tobacco is avoided.

Such smoking substitute systems can form part of nicotine replacement therapies aimed at people who wish to stop smoking and overcome a dependence on nicotine.

Known smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the health risks associated with conventional smoking.

In general, smoking substitute systems are intended to provide a substitute for the rituals of smoking, whilst providing the user with a similar, or improved, experience and satisfaction to those experienced with conventional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach. Some smoking substitute systems are designed to resemble a conventional cigarette and are cylindrical in form with a mouthpiece at one end. Other smoking substitute devices do not generally resemble a cigarette (for example, the smoking substitute device may have a generally box-like form, in whole or in part).

One approach is the so-called "vaping" approach, in which a vaporisable liquid, or an aerosol former, sometimes typically referred to herein as "e-liquid", is heated by a heating device (sometimes referred to herein as an electronic cigarette or "e-cigarette" device) to produce an aerosol vapour which is inhaled by a user. The e-liquid typically includes a base liquid, nicotine and may include a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid and a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically couplable to a consumable including the tank and the heating element. In this way, when the tank of a consumable has been emptied of e-liquid, that consumable is removed from the main body and disposed of. The main body can then be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece and a sealed tank which contains e-liquid. The consumable further includes a heater, which for this device is a heating filament coiled around a portion of a wick. The wick is partially immersed in the e-liquid, and conveys e-liquid from the tank to the heating filament. The system is controlled by a microprocessor on board the main body. The system includes a sensor for detecting when a user is inhaling through the mouthpiece, the microprocessor then activating the device in response. When the system is activated, electrical energy is supplied from the power source to the heating device, which heats e-liquid from the tank to produce a vapour which is inhaled by a user through the mouthpiece.

## *Summary of the Invention*

For a smoking substitute system it is desirable to deliver nicotine into the user's lungs, where it can be absorbed into the bloodstream. However, the present disclosure is based in part on a realisation that some prior art smoking substitute systems, such delivery of nicotine is not efficient. In some prior art systems, the aerosol droplets have a size distribution that is not suitable for delivering nicotine to the lungs. Aerosol droplets of a large particle size tend to be deposited in the mouth and/or upper respiratory tract. Aerosol particles of a small (e.g. sub-micron) particle size can be inhaled into the lungs but may be exhaled without delivering nicotine to the lungs. As a result the user would require drawing a longer puff, more puffs, or vaporising e-liquid with a higher nicotine concentration in order to achieve the desired experience.

Accordingly, there is a need for improvement in the delivery of nicotine to a user in the context of a smoking substitute system.

The present disclosure has been devised in the light of the above considerations.

In a general aspect, embodiments of the present invention relate to a smoking substitute apparatus including one or more adjustable airflow restrictors.

According to a first preferred aspect there is provided a smoking substitute apparatus comprising:

a vaporiser chamber, including a vaporiser configured to vaporise a vaporisable liquid;
a primary airflow path, which passes from a first air inlet of the smoking substitute apparatus through the vaporiser chamber, to an outlet of the smoking substitute apparatus;
a secondary airflow path, which passes from a second air inlet of the smoking substitute apparatus to the outlet of the smoking substitute apparatus, said secondary airflow path bypassing the vaporiser chamber and passing through one or more bypass air ducts;
wherein one or more adjustable airflow restrictors are disposed along either or both of the primary airflow path and the secondary airflow path, said adjustable airflow restrictors being adjustable by a user of the device to vary a draw resistance of the smoking substitute apparatus.

By providing such adjustable airflow restrictors, the inhalation profile (e.g. draw resistance, and other characteristics) can be adjusted to suit the user whilst not distorting the airflow through the vaporiser chamber. Accordingly, advantageous airflow profiles over the vaporiser chamber can be preserved. Moreover, a split or division or airflow between the primary and secondary airflow paths can be modified to cause a preferential airflow through one or other of the primary and secondary airflow paths. For example, if the adjustable airflow restrictor is located along the primary airflow path, air may preferentially flow along the secondary airflow path.

Optionally, the or each airflow restrictor may be a constriction along the respective airflow path, the constriction having an adjustable cross-section. In some examples, the constriction may have an adjustable width.

Advantageously, the or each airflow restrictor may be adjustable through the application of a force, by the user, to a portion of an outer housing of the smoking substitute apparatus.

Conveniently, the or each airflow restrictor may be adjustable through adjustment of a dial attached to a portion of an outer housing of the smoking substitute apparatus.

Optionally, the or each airflow restrictor, which may be disposed along the secondary airflow path, may be located within each of the bypass air ducts.

Conveniently, the or each airflow restrictor, which may be disposed along the primary airflow path, may be located between the first air inlet and a vaporiser chamber inlet of the vaporiser chamber.

Additionally, the smoking substitute apparatus may include two first air inlets, located on respectively opposite sides of the smoking substitute apparatus.

In some embodiments, the smoking substitute apparatus may include two second air inlets, located on respectively opposite sides of the smoking substitute apparatus.

Optionally, the first inlet may provide air to both the primary and secondary airflow path.

Conveniently, the primary airflow path and the secondary airflow path may converge in the outlet of the smoking substitute apparatus. The outlet may be a mouthpiece of the smoking substitute apparatus.

In some embodiments, the primary airflow path and the secondary airflow path may partially overlap, and the or each airflow restrictor may be disposed along the overlapping portion of the airflow paths.

Additionally, the vaporiser chamber may include a flow straightener located between a vaporiser chamber inlet and the vaporiser, which may be configured to induce a laminar airflow over the vaporiser.

Optionally, the vaporiser chamber may include a plenum, located between a vaporiser chamber inlet and the vaporiser, which may be configured to reduce an airflow velocity over the vaporiser.

In some embodiments, the smoking substitute apparatus may include a vaporiser chamber outlet, located between the vaporiser and the outlet of the smoking substitute apparatus, and the vaporiser chamber outlet may be a tapered chimney.

In a second aspect, embodiments of the invention provide a smoking substitute system, including the smoking substitute apparatus of the first aspect (and including any, or any combination insofar as they are compatible, of the optional features disclosed therein) and a main body, the main body including a power source for the vaporiser.

The smoking substitute apparatus may be in the form of a consumable. The consumable may be configured for engagement with a main body. When the consumable is engaged with the main body, the combination of the consumable and the main body may form a smoking substitute system such as a closed smoking substitute system. For example, the consumable may comprise components of the system that are disposable, and the main body may comprise non-disposable or non-consumable components (e.g. power supply, controller, sensor, etc.) that facilitate the generation and/or delivery of aerosol by the consumable. In such an embodiment, the aerosol precursor (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling, e.g. a reservoir of the smoking substitute apparatus, with the aerosol precursor (rather than replacing a consumable component of the apparatus).

In light of this, it should be appreciated that some of the features described herein as being part of the smoking substitute apparatus may alternatively form part of a main body for engagement with the smoking substitute apparatus. This may be the case in particular when the smoking substitute apparatus is in the form of a consumable.

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting, or the like.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol precursor, such as an e-liquid. The e-liquid may, for example, comprise a base liquid. The e-liquid may further comprise nicotine. The base liquid may include propylene glycol and/or vegetable glycerine. The e-liquid may be substantially flavourless. That is, the e-liquid may not contain any deliberately added additional flavourant and may consist solely of a base liquid of propylene glycol and/or vegetable glycerine and nicotine.

The reservoir may be in the form of a tank. At least a portion of the tank may be light-transmissive. For example, the tank may comprise a window to allow a user to visually assess the quantity of e-liquid in the tank. A housing of the smoking substitute apparatus may comprise a corresponding aperture (or slot) or window that may be aligned with a light-transmissive portion (e.g. window) of the tank. The reservoir may be referred to as a "clearomizer" if it includes a window, or a "cartomizer" if it does not.

The smoking substitute apparatus comprises one or more passage for fluid flow therethrough, such as the primary airflow path and secondary airflow path discussed previously. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage, for at least a part of the length of the passage. In this respect, the tank may surround the passage, e.g. in an annular arrangement around the passage.

The smoking substitute apparatus comprises an aerosol or vapour generator (also referred to as a vaporiser). The aerosol generator may comprise a wick. The aerosol generator may further comprise a heater. The wick may comprise a porous material, capable of wicking the aerosol precursor. A portion of the wick may be exposed to airflow in the passage. The wick may also comprise one or more portions in contact with liquid stored in the reservoir. For example, opposing ends of the wick may protrude into the reservoir and an intermediate portion (between the ends) may extend across the passage so as to be exposed to airflow in the passage. Thus, liquid may be drawn (e.g. by capillary action) along the wick, from the reservoir to the portion of the wick exposed to airflow.

The heater may comprise a heating element, which may be in the form of a filament wound about the wick (e.g. the filament may extend helically about the wick in a coil configuration). The heating element may be wound about the intermediate portion of the wick that is exposed to airflow in the passage. The heating element may be electrically connected (or connectable) to a power source. Thus, in operation, the power source may apply a voltage across the heating element so as to heat the heating element by resistive heating. This may cause liquid stored in the wick (i.e. drawn from the tank) to be heated so as to form a vapour and become entrained in air flowing through the passage.

This vapour may subsequently cool to form an aerosol in the passage, typically downstream from the heating element.

The smoking substitute apparatus comprises a vaporisation chamber. The vaporisation chamber may form part of the passage in which the heater is located. The vaporisation chamber may be arranged to be in fluid communication with the inlet and outlet of the passage. The vaporisation chamber may be an enlarged portion of the passage. In this respect, the air as drawn in by the user may entrain the generated vapour in a flow away from heater. The entrained vapour may form an aerosol in the vaporisation chamber, or it may form the aerosol further downstream along the passage. The vaporisation chamber may be at least partially defined by the tank. The tank may substantially (or fully) define the vaporisation chamber. In this respect, the tank may surround the vaporisation chamber, e.g. in an annular arrangement around the vaporisation chamber.

In use, the user may puff on a mouthpiece of the smoking substitute apparatus, i.e. draw on the smoking substitute apparatus by inhaling, to draw in an air stream therethrough. A portion of the air stream (also referred to as a "main airflow") may pass through the vaporisation chamber so as to entrain the vapour generated at the heater. That is, such a main airflow may be heated by the heater (although typically only to a limited extent) as it passes through the vaporisation chamber. In addition, as discussed previously, a portion of the air stream (also referred to as a "dilution airflow" or "bypass airflow")) bypasses the vaporisation chamber and is directed to mix with the generated aerosol downstream from the vaporisation chamber. That is, the dilution airflow may be an air stream at an ambient temperature and may not be directly heated at all by the heater. The dilution airflow may combine with the main airflow for diluting the aerosol contained therein. The dilution airflow may merge with the main airflow along the passage downstream from the vaporisation chamber. Alternatively, the dilution airflow may be directly inhaled by the user without passing though the passage of the smoking substitute apparatus.

As a user puffs on the mouthpiece, vaporised e-liquid entrained in the passing airflow may be drawn towards the outlet of the passage. The vapour may cool, and thereby nucleate and/or condense along the passage to form a plurality of aerosol droplets, e.g. nicotine-containing aerosol droplets. A portion of these aerosol droplets may be delivered to and be absorbed at a target delivery site, e.g. a user's lung, whilst a portion of the aerosol droplets may instead adhere onto other parts of the user's respiratory tract, e.g. the user's oral cavity and/or throat. Typically, in some known smoking substitute apparatuses, the aerosol droplets as measured at the outlet of the passage, e.g. at the mouthpiece, may have a median droplet size, $d_{50}$, of less than $1\mu$m.

The particle droplet size, $d_{50}$, of an aerosol may be measured by a laser diffraction technique. For example, the stream of aerosol output from the outlet of the passage may be drawn through a Malvern Spraytec laser diffraction system, where the intensity and pattern of scattered laser light are analysed to calculate the size and size distribution of aerosol droplets. As will be readily understood, the particle size distribution may be expressed in terms of $d_{10}$, $d_{50}$ and $d_{90}$, for example. Considering a cumulative plot of the volume of the particles measured by the laser diffraction technique, the $d_{10}$ particle size is the particle size below which 10% by volume of the sample lies. The $d_{50}$ particle size is the particle size below which 50% by volume of the sample lies. The $d_{90}$ particle size is the particle size below which 90% by volume of the sample lies. Unless otherwise indicated herein, the particle size measurements are volume-based particle size measurements, rather than number-based or mass-based particle size measurements.

The smoking substitute apparatus (or main body engaged with the smoking substitute apparatus) may comprise a power source. The power source may be electrically connected (or connectable) to a heater of the smoking substitute apparatus (e.g. when the smoking substitute apparatus is engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface of the main body may be configured to transfer electrical power from the power source to a heater of the consumable via the electrical interface of the consumable.

The electrical interface of the smoking substitute apparatus may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an identification means, which may, for example, be in the form of an RFID reader, a barcode or QR code reader. This identification means may be able to identify a characteristic (e.g. a type) of a consumable engaged with the main body. In this respect, the consumable may include any one or more of an RFID chip, a barcode or QR code, or memory within which is an identifier and which can be interrogated via the identification means.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may be provided in the consumable or alternatively may be provided in the main body.

The term "flavourant" is used to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in solid or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The present inventors consider that a flow rate of 1.3 L min$^{-1}$ is towards the lower end of a typical user expectation of flow rate through a conventional cigarette and therefore through a user-acceptable smoking substitute apparatus. The present inventors further consider that a flow rate of 2.0 L min$^{-1}$ is towards the higher end of a typical user expectation of flow rate through a conventional cigarette and therefore through a user-acceptable smoking substitute apparatus. Embodiments of the present invention therefore provide an aerosol with advantageous particle size characteristics across a range of flow rates of air through the apparatus.

The aerosol may have a Dv50 of at least 1.1 $\mu$m, at least 1.2 $\mu$m, at least 1.3 $\mu$m, at least 1.4 $\mu$m, at least 1.5 $\mu$m, at least 1.6 $\mu$m, at least 1.7 $\mu$m, at least 1.8 $\mu$m, at least 1.9 $\mu$m or at least 2.0 $\mu$m.

The aerosol may have a Dv50 of not more than 4.9 $\mu$m, not more than 4.8 $\mu$m, not more than 4.7 $\mu$m, not more than 4.6 $\mu$m, not more than 4.5 $\mu$m, not more than 4.4 $\mu$m, not more than 4.3 $\mu$m, not more than 4.2 $\mu$m, not more than 4.1 $\mu$m, not more than 4.0 $\mu$m, not more than 3.9 $\mu$m, not more than 3.8 $\mu$m, not more than 3.7 $\mu$m, not more than 3.6 $\mu$m, not more than 3.5 $\mu$m, not more than 3.4 $\mu$m, not more than 3.3 $\mu$m, not more than 3.2 $\mu$m, not more than 3.1 $\mu$m or not more than 3.0 $\mu$m.

A particularly preferred range for Dv50 of the aerosol is in the range 2-3 $\mu$m.

The air inlet, flow passage, outlet and the vaporisation chamber may be configured so that, when the air flow rate inhaled by the user through the apparatus is 1.3 L min$^{-1}$, the average magnitude of velocity of air in the vaporisation chamber is in the range 0-1.3 ms$^{-1}$. The average magnitude velocity of air may be calculated based on knowledge of the geometry of the vaporisation chamber and the flow rate.

When the air flow rate inhaled by the user through the apparatus is 1.3 L min$^{-1}$, the average magnitude of velocity of air in the vaporisation chamber may be at least 0.001 ms$^{-1}$, or at least 0.005 ms$^{-1}$, or at least 0.01 ms$^{-1}$, or at least 0.05 ms$^{-1}$.

When the air flow rate inhaled by the user through the apparatus is 1.3 L min$^{-1}$, the average magnitude of velocity of air in the vaporisation chamber may be at most 1.2 ms$^{-1}$, at most 1.1 ms$^{-1}$, at most 1.0 ms$^{-1}$, at most 0.9 ms$^{-1}$, at most 0.8 ms$^{-1}$, at most 0.7 ms$^{-1}$ or at most 0.6 ms$^{-1}$.

The air inlet, flow passage, outlet and the vaporisation chamber may be configured so that, when the air flow rate inhaled by the user through the apparatus is 2.0 L min$^{-1}$, the average magnitude of velocity of air in the vaporisation chamber is in the range 0-1.3 ms$^{-1}$. The average magnitude velocity of air may be calculated based on knowledge of the geometry of the vaporisation chamber and the flow rate.

When the air flow rate inhaled by the user through the apparatus is 2.0 L min$^{-1}$, the average magnitude of velocity of air in the vaporisation chamber may be at least 0.001 ms$^{-1}$, or at least 0.005 ms$^{-1}$, or at least 0.01 ms$^{-1}$, or at least 0.05 ms$^{-1}$.

When the air flow rate inhaled by the user through the apparatus is 2.0 L min$^{-1}$, the average magnitude of velocity of air in the vaporisation chamber may be at most 1.2 ms$^{-1}$, at most 1.1 ms$^{-1}$, at most 1.0 ms$^{-1}$, at most 0.9 ms$^{-1}$, at most 0.8 ms$^{-1}$, at most 0.7 ms$^{-1}$ or at most 0.6 ms$^{-1}$.

When the calculated average magnitude of velocity of air in the vaporisation chamber is in the ranges specified, it is considered that the resultant aerosol particle size is advantageously controlled to be in a desirable range. It is further considered that the configuration of the apparatus can be selected so that the average magnitude of velocity of air in the vaporisation chamber can be brought within the ranges specified, at the exemplary flow rate of 1.3 L min$^{-1}$ and/or

the exemplary flow rate of 2.0 L min⁻¹.

The aerosol generator may comprise a vaporiser element loaded with aerosol precursor, the vaporiser element being heatable by a heater and presenting a vaporiser element surface to air in the vaporisation chamber. A vaporiser element region may be defined as a volume extending outwardly from the vaporiser element surface to a distance of 1 mm from the vaporiser element surface.

The air inlet, flow passage, outlet and the vaporisation chamber may be configured so that, when the air flow rate inhaled by the user through the apparatus is 1.3 L min⁻¹, the average magnitude of velocity of air in the vaporiser element region is in the range 0-1.2 ms⁻¹. The average magnitude of velocity of air in the vaporiser element region may be calculated using computational fluid dynamics.

When the air flow rate inhaled by the user through the apparatus is 1.3 L min⁻¹, the average magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the average magnitude of velocity of air in the vaporiser element region may be at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

The air inlet, flow passage, outlet and the vaporisation chamber may be configured so that, when the air flow rate inhaled by the user through the apparatus is 2.0 L min⁻¹, the average magnitude of velocity of air in the vaporiser element region is in the range 0-1.2 ms⁻¹. The average magnitude of velocity of air in the vaporiser element region may be calculated using computational fluid dynamics.

When the air flow rate inhaled by the user through the apparatus is 2.0 L min⁻¹, the average magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 2.0 L min⁻¹, the average magnitude of velocity of air in the vaporiser element region may be at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

When the average magnitude of velocity of air in the vaporiser element region is in the ranges specified, it is considered that the resultant aerosol particle size is advantageously controlled to be in a desirable range. It is further considered that the velocity of air in the vaporiser element region is more relevant to the resultant particle size characteristics than consideration of the velocity in the vaporisation chamber as a whole. This is in view of the significant effect of the velocity of air in the vaporiser element region on the cooling of the vapour emitted from the vaporiser element surface.

Additionally or alternatively is it relevant to consider the maximum magnitude of velocity of air in the vaporiser element region.

Therefore, the air inlet, flow passage, outlet and the vaporisation chamber may be configured so that, when the air flow rate inhaled by the user through the apparatus is 1.3 L min⁻¹, the maximum magnitude of velocity of air in the vaporiser element region is in the range 0-2.0 ms⁻¹.

When the air flow rate inhaled by the user through the apparatus is 1.3 L min⁻¹, the maximum magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the maximum magnitude of velocity of air in the vaporiser element region may be at most 1.9 ms⁻¹, at most 1.8 ms⁻¹, at most 1.7 ms⁻¹, at most 1.6 ms⁻¹, at most 1.5 ms⁻¹, at most 1.4 ms⁻¹, at most 1.3 ms⁻¹ or at most 1.2 ms⁻¹.

The air inlet, flow passage, outlet and the vaporisation chamber may be configured so that, when the air flow rate inhaled by the user through the apparatus is 2.0 L min⁻¹, the maximum magnitude of velocity of air in the vaporiser element region is in the range 0-2.0 ms⁻¹.

When the air flow rate inhaled by the user through the apparatus is 2.0 L min⁻¹, the maximum magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 2.0 L min⁻¹, the maximum magnitude of velocity of air in the vaporiser element region may be at most 1.9 ms⁻¹, at most 1.8 ms⁻¹, at most 1.7 ms⁻¹, at most 1.6 ms⁻¹, at most 1.5 ms⁻¹, at most 1.4 ms⁻¹, at most 1.3 ms⁻¹ or at most 1.2 ms⁻¹.

It is considered that configuring the apparatus in a manner to permit such control of velocity of the airflow at the vaporiser permits the generation of aerosols with particularly advantageous particle size characteristics, including Dv50 values.

Additionally or alternatively is it relevant to consider the turbulence intensity in the vaporiser chamber in view of the effect of turbulence on the particle size of the generated aerosol. For example, the air inlet, flow passage, outlet and the vaporisation chamber may be configured so that, when the air flow rate inhaled by the user through the apparatus is 1.3 L min⁻¹, the turbulence intensity in the vaporiser element region is not more than 1%.

When the air flow rate inhaled by the user through the apparatus is 1.3 L min⁻¹, the turbulence intensity in the vaporiser element region may be not more than 0.95%, not more than 0.9%, not more than 0.85%, not more than 0.8%, not more than 0.75%, not more than 0.7%, not more than 0.65% or not more than 0.6%.

It is considered that configuring the apparatus in a manner to permit such control of the turbulence intensity in the vaporiser element region permits the generation of aerosols with particularly advantageous particle size characteristics, including Dv50 values.

Following detailed investigations, the inventors consider, without wishing to be bound by theory, that the particle size characteristics of the generated aerosol may be determined by the cooling rate experienced by the vapour after emission from the vaporiser element (e.g. wick). In particular, it appears that imposing a relatively slow cooling rate on the vapour has the effect of generating aerosols with a relatively large particle size. The parameters discussed above (velocity and turbulence intensity) are considered to be mechanisms for implementing a particular cooling dynamic to the vapour.

More generally, it is considered that the air inlet, flow passage, outlet and the vaporisation chamber may be configured so that a desired cooling rate is imposed on the vapour. The particular cooling rate to be used depends of course on the nature of the aerosol precursor and other conditions. However, for a particular aerosol precursor it is possible to define a set of testing conditions in order to define the cooling rate, and by extension this imposes limitations on the configuration of the apparatus to permit such cooling rates as are shown to result in advantageous aerosols. Accordingly, the air inlet, flow passage, outlet and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min$^{-1}$.

Additionally or alternatively, the air inlet, flow passage, outlet and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min$^{-1}$.

Cooling of the vapour such that the time taken to cool to 50 °C is not less than 16 ms corresponds to an equivalent linear cooling rate of not more than 10 °C/ms.

The equivalent linear cooling rate of the vapour to 50 °C may be not more than 9 °C/ms, not more than 8 °C/ms, not more than 7 °C/ms, not more than 6 °C/ms or not more than 5 °C/ms.

Cooling of the vapour such that the time taken to cool to 50 °C is not less than 32 ms corresponds to an equivalent linear cooling rate of not more than 5 °C/ms.

The testing protocol set out above considers the cooling of the vapour (and subsequent aerosol) to a temperature of 50 °C. This is a temperature which can be considered to be suitable for an aerosol to exit the apparatus for inhalation by a user without causing significant discomfort. It is also possible to consider cooling of the vapour (and subsequent aerosol) to a temperature of 75 °C. Although this temperature is possibly too high for comfortable inhalation, it is considered that the particle size characteristics of the aerosol are substantially settled by the time the aerosol cools to this temperature (and they may be settled at still higher temperature).

Accordingly, the air inlet, flow passage, outlet and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min$^{-1}$.

Additionally or alternatively, the air inlet, flow passage, outlet and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min$^{-1}$.

Cooling of the vapour such that the time taken to cool to 75 °C is not less than 4.5 ms corresponds to an equivalent linear cooling rate of not more than 30 °C/ms.

The equivalent linear cooling rate of the vapour to 75 °C may be not more than 29 °C/ms, not more than 28 °C/ms, not more than 27 °C/ms, not more than 26 °C/ms, not more than 25 °C/ms, not more than 24 °C/ms, not more than 23 °C/ms, not more than 22 °C/ms, not more than 21 °C/ms, not more than 20 °C/ms, not more than 19 °C/ms, not more than 18 °C/ms, not more than 17 °C/ms, not more than 16 °C/ms, not more than 15 °C/ms, not more than 14 °C/ms, not more than 13 °C/ms, not more than 12 °C/ms, not more than 11 °C/ms or not more than 10 °C/ms.

Cooling of the vapour such that the time taken to cool to 75 °C is not less than 13 ms corresponds to an equivalent linear cooling rate of not more than 10 °C/ms.

It is considered that configuring the apparatus in a manner to permit such control of the cooling rate of the vapour permits the generation of aerosols with particularly advantageous particle size characteristics, including Dv50 values.

The invention includes the combination of the aspects and preferred features described except where such a combination is clearly impermissible or expressly avoided.

### *Summary of the Figures*

So that the invention may be understood, and so that further aspects and features thereof may be appreciated, embodiments illustrating the principles of the invention will now be discussed in further detail with reference to the accompanying figures, in which:

Figure 1 illustrates a set of rectangular tubes for use in experiments to assess the effect of flow and cooling conditions at the wick on aerosol properties. Each tube has the same depth and length but different width.

Figure 2 shows a schematic perspective longitudinal cross sectional view of an example rectangular tube with a wick and heater coil installed.

Figure 3 shows a schematic transverse cross sectional view an example rectangular tube with a wick and heater coil installed. In this example, the internal width of the tube is 12 mm.

Figures 4A-4D show air flow streamlines in the four devices used in a turbulence study.

Figure 5 shows the experimental set up to investigate the influence of inflow air temperature on aerosol particle size, in order to investigate the effect of vapour cooling rate on aerosol generation.

Figure 6 shows a schematic longitudinal cross sectional view of a first smoking substitute apparatus (pod 1) used to assess influence of inflow air temperature on aerosol particle size.

Figure 7 shows a schematic longitudinal cross sectional view of a second smoking substitute apparatus (pod 2) used to assess influence of inflow air temperature on aerosol particle size.

Figure 8A shows a schematic longitudinal cross sectional view of a third smoking substitute apparatus (pod 3) used to assess influence of inflow air temperature on aerosol particle size. Figure 8B shows a schematic longitudinal cross sectional view of the same third smoking substitute apparatus (pod 3) in a direction orthogonal to the view taken in Figure 8A.

Figure 9 shows a plot of aerosol particle size (Dv50) experimental results against calculated air velocity.

Figure 10 shows a plot of aerosol particle size (Dv50) experimental results against the flow rate through the apparatus for a calculated air velocity of 1 m/s.

Figure 11 shows a plot of aerosol particle size (Dv50) experimental results against the average magnitude of the velocity in the vaporiser surface region, as obtained from CFD modelling.

Figure 12 shows a plot of aerosol particle size (Dv50) experimental results against the maximum magnitude of the velocity in the vaporiser surface region, as obtained from CFD modelling.

Figure 13 shows a plot of aerosol particle size (Dv50) experimental results against the turbulence intensity.

Figure 14 shows a plot of aerosol particle size (Dv50) experimental results dependent on the temperature of the air and the heating state of the apparatus.

Figure 15 shows a plot of aerosol particle size (Dv50) experimental results against vapour cooling rate to 50°C.

Figure 16 shows a plot of aerosol particle size (Dv50) experimental results against vapour cooling rate to 75°C.

Figure 17 is a schematic front view of a smoking substitute system, according to a first embodiment, in an engaged position;

Figure 18 is a schematic front view of the smoking substitute system of the first embodiment in a disengaged position;

Figure 19 is a schematic longitudinal cross sectional view of a smoking substitute apparatus of a reference arrangement; and

Figure 20 is an enlarged schematic cross sectional view of part of the air passage and vaporisation chamber of the first embodiment;

Figure 21 is a schematic longitudinal cross sectional view of a smoking substitute apparatus of the first embodiment;

Figure 22 is a schematic longitudinal cross sectional view of a smoking substitute apparatus of a second embodiment; and

Figure 23 is a schematic longitudinal cross sectional view of a smoking substitute apparatus of a third embodiment.

## Detailed Description of the Invention

Further background to the present invention and further aspects and embodiments of the present invention will now be discussed with reference to the accompanying figures. Further aspects and embodiments will be apparent to those skilled in the art. The contents of all documents mentioned in this text are incorporated herein by reference in their entirety.

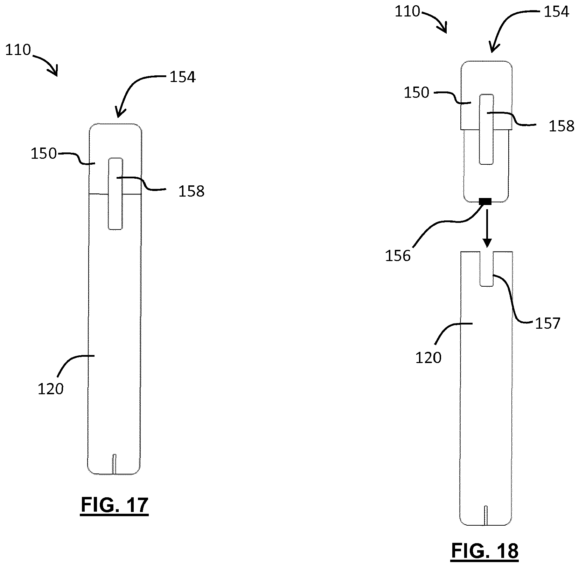

Figures 17 and 18 illustrate a smoking substitute system in the form of an e-cigarette system 110. The system 110 comprises a main body 120 of the system 110, and a smoking substitute apparatus in the form of an e-cigarette consumable (or "pod") 150. In the illustrated embodiment the consumable 150 (sometimes referred to herein as a smoking substitute apparatus) is removable from the main body 120, so as to be a replaceable component of the system 110. The e-cigarette system 110 is a closed system in the sense that it is not intended that the consumable should be refillable with e-liquid by a user.

As is apparent from Figures 17 and 18, the consumable 150 is configured to engage the main body 120. Figure 17 shows the main body 120 and the consumable 150 in an engaged state, whilst Figure 18 shows the main body 120 and the consumable 150 in a disengaged state. When engaged, a portion of the consumable 150 is received in a cavity of corresponding shape in the main body 120 and is retained in the engaged position by way of a snap-engagement mechanism. In other embodiments, the main body 120 and consumable 150 may be engaged by screwing one into (or onto) the other, or through a bayonet fitting, or by way of an interference fit.

The system 110 is configured to vaporise an aerosol precursor, which in the illustrated embodiment is in the form of a nicotine-based e-liquid 160. The e-liquid 160 comprises nicotine and a base liquid including propylene glycol and/or vegetable glycerine. In the present embodiment, the e-liquid 160 is flavoured by a flavourant. In other embodiments, the e-liquid 160 may be flavourless and thus may not include any added flavourant.

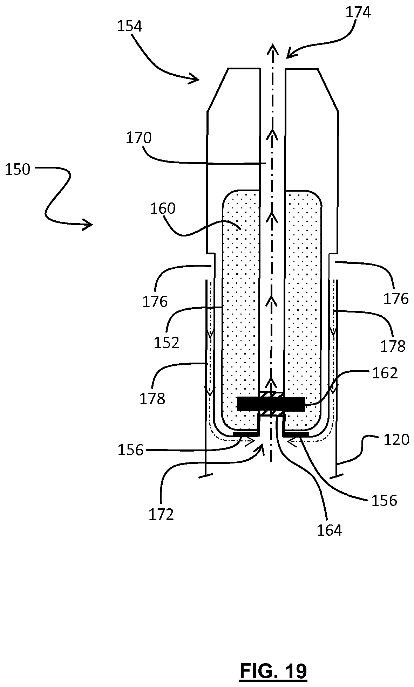

Figure 19 shows a schematic longitudinal cross sectional view of a reference smoking substitute apparatus which may form a part of the smoking substitute system shown in Figures 17 and 18. Whilst not itself forming an embodiment of the invention, features of the apparatus shown in Figure 19 are applicable with various embodiments of the invention. In Figure 19, the e-liquid 160 is stored within a reservoir in the form of a tank 152 that forms part of the consumable 150. In the example shown, the consumable 150 is a "single-use" consumable 150. That is, upon exhausting the e-liquid 160 in the tank 152, the intention is that the user disposes of the entire consumable 150. The term "single-use" does not necessarily mean the consumable is designed to be disposed of after a single smoking session. Rather, it defines the consumable 150 is not arranged to be refilled after the e-liquid contained in the tank 152 is depleted. The tank may include a vent (not shown) to allow ingress of air to replace e-liquid that has been used from the tank. The consumable 150 preferably includes a window 158 (see Figures 17 and 18), so that the amount of e-liquid in the tank 152 can be visually assessed. The main body 120 includes a slot 157 so that the window 158 of the consumable 150 can be seen whilst the rest of the tank 152 is obscured from view when the consumable 150 is received in the cavity of the main body 120. The consumable 150 may be referred to as a "clearomizer" when it includes a window 158, or a "cartomizer" when it does not.

In embodiments of the invention, the e-liquid (i.e. aerosol precursor) may be the only part of the system that is truly "single-use". That is, the tank may be refillable with e-liquid or the e-liquid may be stored in a non-consumable component of the system. For example, in such other embodiments, the e-liquid may be stored in a tank located in the main body or stored in another component that is itself not single-use (e.g. a refillable cartomizer).

The external wall of tank 152 is provided by a casing of the consumable 150. The tank 152 annularly surrounds, and thus defines a portion of, a passage 170 that extends between a vaporiser inlet 172 and an outlet 174 at opposing ends of the consumable 150. In this respect, the passage 170 comprises an upstream end at the end of the consumable 150 that engages with the main body 120, and a downstream end at an opposing end of the consumable 150 that comprises a mouthpiece 154 of the system 110.

When the consumable 150 is received in the cavity of the main body 120 as shown in Figure 19, a plurality of device air inlets 176 are formed at the boundary between the casing of the consumable and the casing of the main body. The device air inlets 176 are in fluid communication with the vaporiser inlet 172 through an inlet flow channel 178 formed in the cavity of the main body which is of corresponding shape to receive a part of the consumable 150. Air from outside of the system 110 can therefore be drawn into the passage 170 through the device air inlets 176 and the inlet flow channels 178.

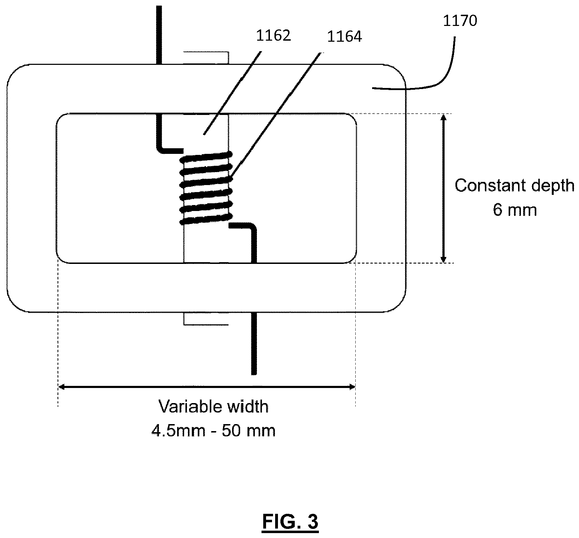

When the consumable 150 is engaged with the main body 120, a user can inhale (i.e. take a puff) via the mouthpiece 154 so as to draw air through the passage 170, and so as to form an airflow (indicated by the dashed arrows in Figure 3) in a direction from the vaporiser inlet 172 to the outlet 174. Although not illustrated, the passage 170 may be partially defined by a tube (e.g. a metal tube) extending through the consumable 150. In Figure 3, for simplicity, the passage 170 is shown with a substantially circular cross-sectional profile with a constant diameter along its length. In embodiments of the invention, the passage may have other cross-sectional profiles, such as oval shaped or polygonal shaped profiles. Further, in other embodiments, the cross sectional profile and the diameter (or hydraulic diameter) of the passage may vary along its longitudinal axis.

The smoking substitute system 110 is configured to vaporise the e-liquid 160 for inhalation by a user. To provide this operability, the consumable 150 comprises a heater having a porous wick 162 and a resistive heating element in the form of a heating filament 164 that is helically wound (in the form of a coil) around a portion of the porous wick 162. The porous wick 162 extends across the passage 170 (i.e. transverse to a longitudinal axis of the passage 170 and thus also transverse to the airflow along the passage 170 during use) and opposing ends of the wick 162 extend into the tank 152 (so as to be immersed in the e-liquid 160). In this way, e-liquid 160 contained in the tank 152 is conveyed from the opposing ends of the porous wick 162 to a central portion of the porous wick 162 so as to be exposed to the airflow in the passage 170.

The helical filament 164 is wound about the exposed central portion of the porous wick 162 and is electrically connected to an electrical interface in the form of electrical contacts 156 mounted at the end of the consumable that is proximate the main body 120 (when the consumable and the main body are engaged). When the consumable 150 is engaged with the main body 120, electrical contacts 156 make contact with corresponding electrical contacts (not shown) of the main body 120. The main body electrical contacts are electrically connectable to a power source (not shown) of the main body 120, such that (in the engaged position) the filament 164 is electrically connectable to the power source. In this way, power can be supplied by the main body 120 to the filament 164 in order to heat the filament 164. This heats the porous wick 162 which causes e-liquid 160 conveyed by the porous wick 162 to vaporise and thus to be released from the porous wick 162. The vaporised e-liquid becomes entrained in the airflow and, as it cools in the airflow (between the heated wick and the outlet 174 of the passage 170), condenses to form an aerosol. This aerosol is then inhaled, via the mouthpiece 154, by a user of the system 110. As e-liquid is lost from the heated portion of the wick, further e-liquid is drawn along the wick from the tank to replace the e-liquid lost from the heated portion of the wick.

The filament 164 and the exposed central portion of the porous wick 162 are positioned across the passage 170. More specifically, the part of passage that contains the filament 164 and the exposed portion of the porous wick 162 forms a vaporisation chamber. In the illustrated example, the vaporisation chamber has the same cross-sectional diameter as the passage 170. However, in other embodiments the vaporisation chamber may have a different cross sectional profile as the passage 170. For example, the vaporisation chamber may have a larger cross sectional diameter than at least some of the downstream part of the passage 170 so as to enable a longer residence time for the air inside the vaporisation chamber.

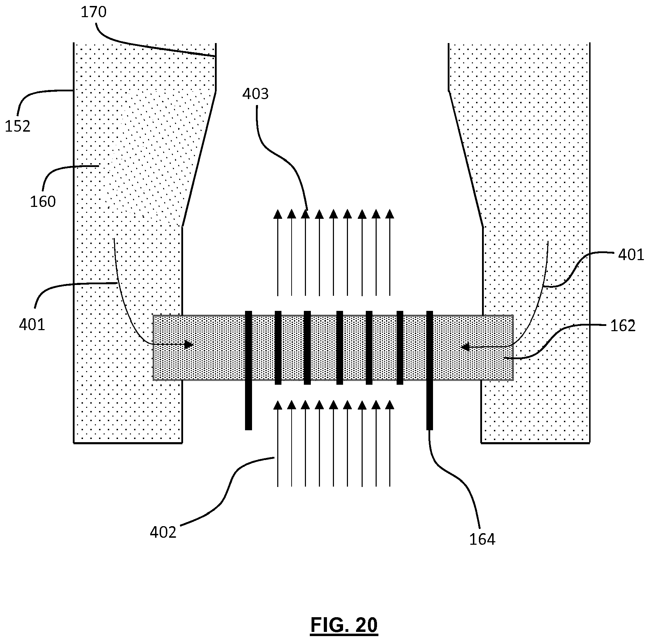

Figure 20 illustrates in more detail the vaporisation chamber as present in embodiments of the invention, and therefore the region of the consumable 150 around the wick 162 and filament 164. The helical filament 164 is wound around a central portion of the porous wick 162. The porous wick extends across passage 170. E-liquid 160 contained within the tank 152 is conveyed as illustrated schematically by arrows 401, i.e. from the tank and towards the central portion of the porous wick 162.

When the user inhales, air 402 is drawn from through the inlets 176 shown in Figure 19, along inlet flow channel 178 to vaporisation chamber inlet 172 and into the vaporisation chamber containing porous wick 162. The porous wick 162 extends substantially transverse to the airflow direction. The airflow passes around the porous wick, at least a portion of the airflow substantially following the surface of the porous wick 162. In examples where the porous wick has a cylindrical cross-sectional profile, the airflow may follow a curved path around an outer periphery of the porous wick 162.

At substantially the same time as the airflow passes around the porous wick 162, the filament 164 is heated so as to vaporise the e-liquid which has been wicked into the porous wick. The airflow passing around the porous wick 162 picks up this vaporised e-liquid, and the vapour-containing airflow 403 is drawn in direction 403 further down passage 170.

The power source of the main body 120 may be in the form of a battery (e.g. a rechargeable battery such as a lithium ion battery). The main body 120 may comprise a connector in the form of e.g. a USB port for recharging this battery. The main body 120 may also comprise a controller that controls the supply of power from the power source to the main body electrical contacts (and thus to the filament 164). That is, the controller may be configured to control a voltage applied across the main body electrical contacts, and thus the voltage applied across the filament 164. In this way, the filament 164 may only be heated under certain conditions (e.g. during a puff and/or only when the system is in an active state). In this respect, the main body 120 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 120 and consumable 150 may comprise a further interface which may, for example, be in the form of an RFID reader, a barcode or QR code reader. This interface may be able to identify a characteristic (e.g. a type) of a consumable 150 engaged with the main body 120. In this respect, the consumable 150 may include any one or more of an RFID chip, a barcode or QR code, or memory within which is an identifier and which can be interrogated via the interface.

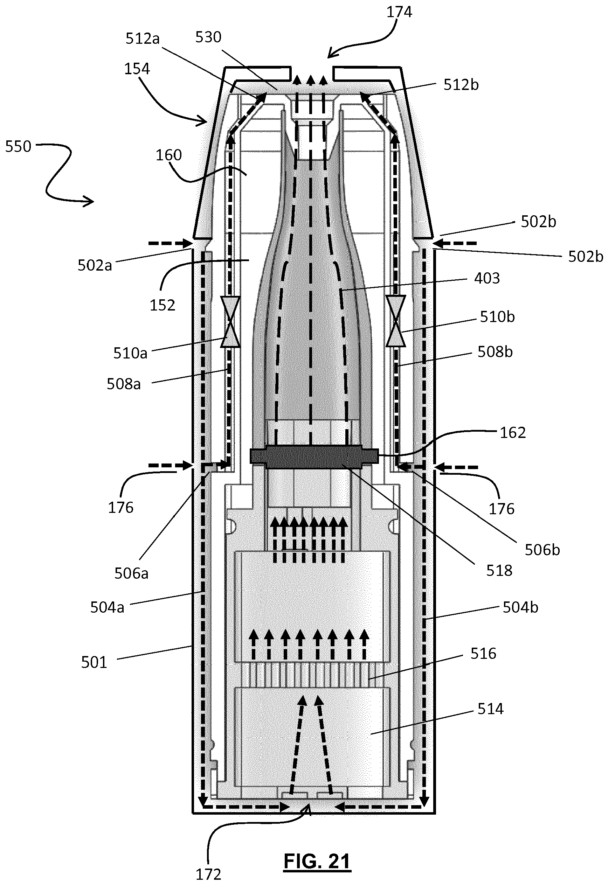

Figure 21 shows a schematic longitudinal cross sectional view of a smoking substitute apparatus 550 according to the first embodiment. It shares a number of features with the reference arrangement shown in Figure 19, and so like features are indicated by like reference numerals. The smoking substitute apparatus has a housing 501, within which the majority of its components are contained. Holes within the housing provide the primary air inlets 176 and secondary air inlets 502a/502b. In this example, the secondary air inlets 502a/502b, located on either side of the smoking substitute apparatus, are closer to the mouthpiece 154 than the primary air inlets 176.

The primary air inlets 176 and secondary air inlets 502a/b are fluidly connected to shared airflow paths 504a/b. These shared airflow paths extend along an inside of the smoking substitute apparatus 550 to the vaporisation chamber inlet 172. The shared airflow paths are an example of the primary airflow path referred to previously. Also fluidly connected to the primary air inlets 176 and secondary air inlets 502a/b are bypass air inlets 506a and 506b (one on either side of the smoking substitute apparatus). The bypass air inlets connect to bypass air ducts 508a and 508b respectively. Each of the bypass air duct contains an adjustable airflow restrictor 510a / 510b. These adjustable airflow restrictors allow a user to tune or adjust the draw resistance of the smoking substitute apparatus in a manner which does not (at least directly) affect the airflow through the vaporisation chamber inlet 172. The bypass airflow ducts 508a/b end at bypass air duct outlets 512a / 512b which are located proximal to the smoking substitute apparatus outlet 174.

Also shown in Figure 21 are further features of the vaporisation chamber located downstream of the vaporiser chamber inlet 172. After passing through the inlet, the airflow expands into plenum 514. The plenum functions to slow the velocity of the air which is flowing towards the vaporiser. Between the plenum and vaporiser 518, which is a coil and wick arrangement, is a flow straightener 516. The flow straightener in this example is formed from an array of tubes having a cylindrical axis aligned with a flow direction towards the coil and wick arrangement. The flow straightener can also be provided as a mesh.

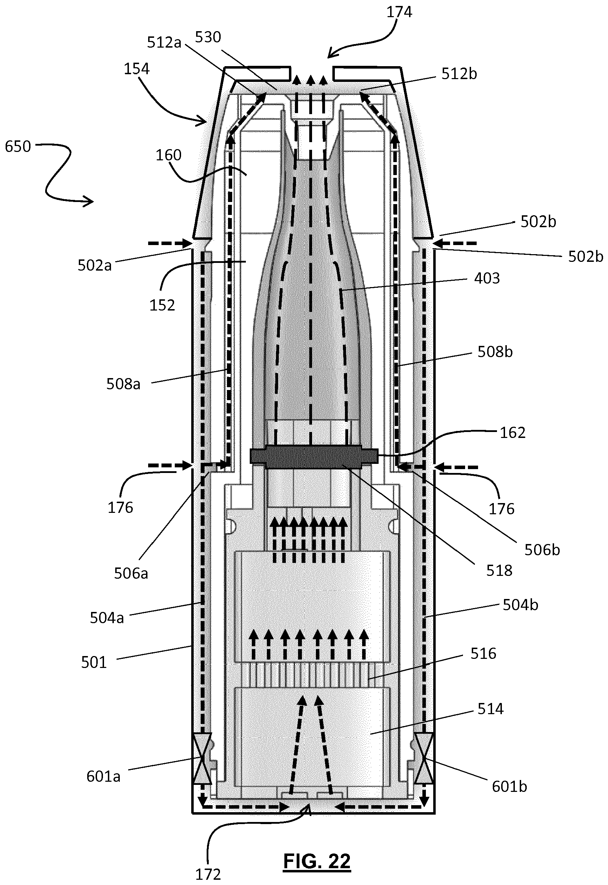

Figure 22 shows a smoking substitute apparatus 600 according to second embodiment. It shares a number of features with the first embodiment, and so like features are indicated by like reference numerals. In contrast to Figure 21, the adjustable flow restrictors 601a and 601b are located within the shared airflow paths 504a and 504b. This allows the user to tune or adjust the draw resistance of the smoking substitute apparatus which also influences the air flow properties over and around the vaporiser. For example, by decreasing the draw resistance, the user may be able to draw air through the vaporiser and a higher velocity which may affect the particle size distribution of the aerosol generated therefrom.

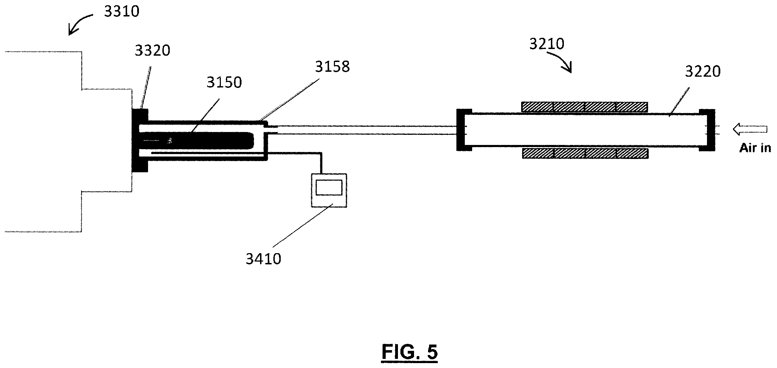

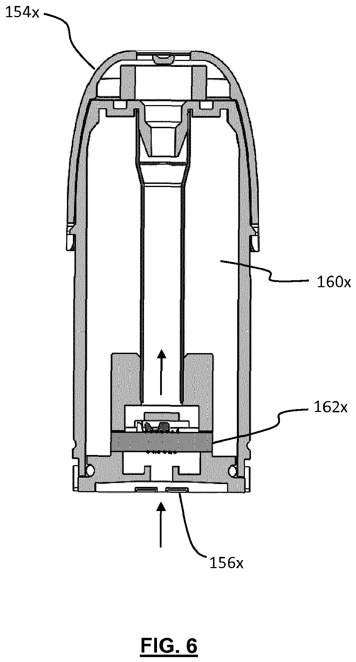

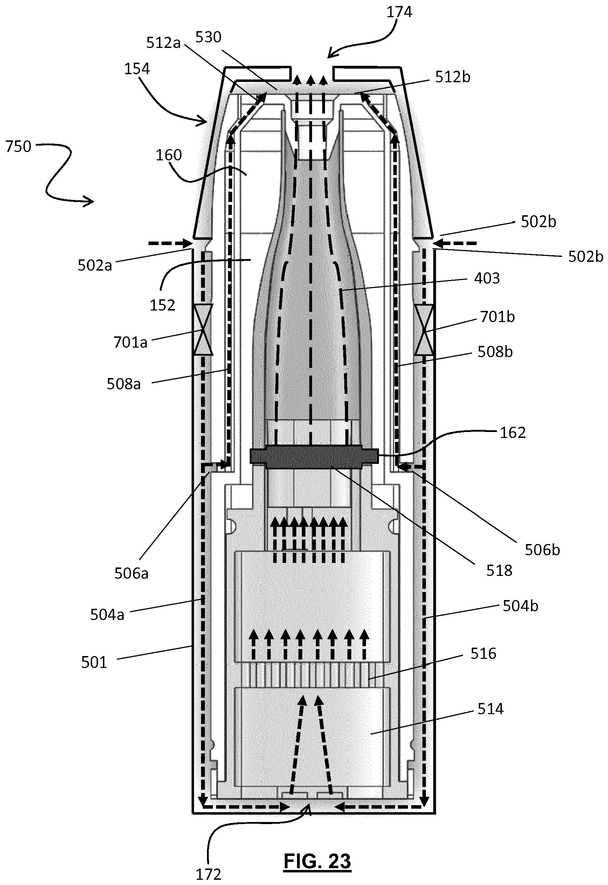

Figure 23 shows a smoking substitute apparatus 750 according to a third embodiment. It shares a number of features with the first and second embodiments, and so like features are indicated by like reference numerals. In contrast to Figures 5 and 6, the smoking substitute apparatus 750 of Figure 23 has only one air inlet 502a/502b located in an outer housing of the smoking substitute apparatus. Therefore the shared airflow path extends from the air inlet 502a/502b to the bypass airflow inlet 506a/b, before separating. In this example then, bypass airflow inlet(s) 506a/b provide the second air inlet(s) into the secondary airflow path. A further difference is that the adjustable airflow restrictor 701a / 701b is located in the shared airflow path located between the air inlet 502a/502b and the bypass airflow inlet. This allows the user to tune or adjust the draw resistance of the smoking substitute apparatus in a manner which affects the airflow through both the bypass airflow ducts 508a/508b and the vaporiser chamber.

There now follows a disclosure of certain experimental work undertaken to determine the effects of certain conditions in the smoking substitute apparatus on the particle size of the generated aerosol.

This work is relevant in particular in view of the approach taken by the embodiments of the invention to control the flow conditions at the wick.

1. Introduction

Aerosol droplet size is a considered to be an important characteristic for smoking substitution devices. Droplets in the range of 2-5 $\mu$m are preferred in order to achieve improved nicotine delivery efficiency and to minimise the hazard of second-hand smoking. However, at the time of writing (September 2019), commercial EVP devices typically deliver aerosols with droplet size averaged around 0.5 $\mu$m, and to the knowledge of the inventors not a single commercially available device can deliver an aerosol with an average particle size exceeding 1 $\mu$m.

The present inventors speculate, without themselves wishing to be bound by theory, that there has to date been a lack of understanding in the mechanisms of e-liquid evaporation, nucleation and droplet growth in the context of aerosol generation in smoking substitute devices. The present inventors have therefore studied these issues in order to provide insight into mechanisms for the generation of aerosols with larger particles. The present inventors have carried out experimental and modelling work alongside theoretical investigations, leading to significant achievements as now reported.

This disclosure considers the roles of air velocity, air turbulence and vapour cooling rate in affecting aerosol particle size.

2. Experiments

In this work, a Malvern PANalytical Spraytec laser diffraction system was employed for the particle size measurement. In order to limit the number of variables, the same coil and wick (1.5 ohms Ni-Cr coil, 1.8 mm Y07 cotton wick), the same e-liquid (1.6% freebase nicotine, 65:35 propylene glycol (PG)/vegetable glycerine (VG) ratio, no added flavour) and the same input power (10W) were used in all experiments. Y07 represents the grade of cotton wick, meaning that the cotton has a linear density of 0.7 grams per meter.

Particle sizes were measured in accordance with ISO 13320:2009(E), which is an international standard on laser diffraction methods for particle size analysis. This is particularly well suited to aerosols, because there is an assumption in this standard that the particles are spherical (which is a good assumption for liquid-based aerosols). The standard is stated to be suitable for particle sizes in the range 0.1 micron to 3 mm.

The results presented here concentrate on the volume-based median particle size Dv50. This is to be taken to be the same as the parameter $d_{50}$ used above.

2.1. Rectangular tube testing

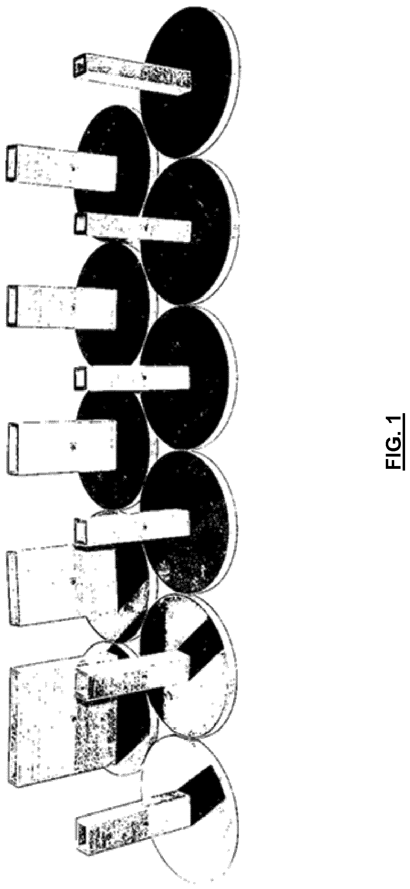

The work reported here based on the inventors' insight that aerosol particle size might be related to: 1) air velocity; 2) flow rate; and 3) Reynolds number. In a given EVP device, these three parameters are interlinked to each other, making it difficult to draw conclusions on the roles of each individual factor. In order to decouple these factors, experiments were carried out using a set of rectangular tubes having different dimensions. These were manufactured by 3D printing. The rectangular tubes were 3D printed in an MJP 2500 3D printer. Figure 1 illustrates the set of rectangular tubes. Each tube has the same depth and length but different width. Each tube has an integral end plate in order to provide a seal against air flow outside the tube. Each tube also has holes formed in opposing side walls in order to accommodate a wick.

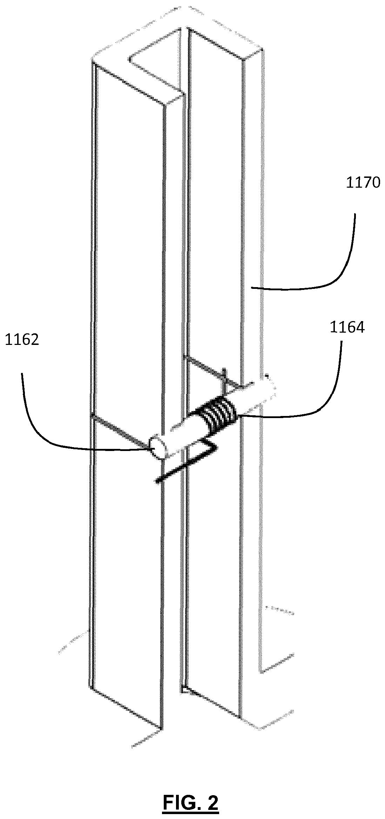

Figure 2 shows a schematic perspective longitudinal cross sectional view of an example rectangular tube 1170 with a wick 1162 and heater coil 1164 installed. The location of the wick is about half way along the length of the tube. This is intended to allow the flow of air along the tube to settle before reaching the wick.

Figure 3 shows a schematic transverse cross sectional view an example rectangular tube 1170 with a wick 1162 and heater coil 1164 installed. In this example, the internal width of the tube is 12 mm

The rectangular tubes were manufactured to have same internal depth of 6 mm in order to accommodate the standardized coil and wick, however the tube internal width varied from 4.5 mm to 50 mm. In this disclosure, the "tube size" is referred to as the internal width of rectangular tubes.

The rectangular tubes with different dimensions were used to generate aerosols that were tested for particle size in a Malvern PANalytical Spraytec laser diffraction system. An external digital power supply was dialled to 2.6A constant current to supply 10W power to the heater coil in all experiments. Between two runs, the wick was saturated manually by applying one drop of e-liquid on each side of the wick.

Three groups of experiments were carried out in this study:

1. 1.3 lpm (litres per minute, L min⁻¹ or LPM) constant flow rate on different size tubes
2. 2.0 lpm constant flow rate on different size tubes
3. 1 m/s constant air velocity on 3 tubes: i) 5mm tube at 1.4 lpm flow rate; ii) 8mm tube at 2.8 lpm flow rate; and iii) 20mm tube at 8.6 lpm flow rate.

Table 1 shows a list of experiments in this study. The values in "calculated air velocity" column were obtained by simply dividing the flow rate by the intersection area at the centre plane of wick. Reynolds numbers (Re) were calculated through the following equation:

$$Re = \frac{\rho v L}{\mu}$$

where: $\rho$ is the density of air (1.225 kg/m$^3$); $v$ is the calculated air velocity in table 1; $\mu$ is the viscosity of air (1.48 × 10$^{-5}$ m$^2$/s); $L$ is the characteristic length calculated by:

$$L = \frac{4P}{A}$$

where: $P$ is the perimeter of the flow path's intersection, and $A$ is the area of the flow path's intersection.

*Table 1. List of experiments in the rectangular tube study*

|  | Tube size [mm] | Flow rate [lpm] | Reynolds number | Calculated air velocity [m/s] |
|---|---|---|---|---|
| 1.3 lpm constant flow rate | 4.5 | 1.3 | 153 | 1.17 |
|  | 6 | 1.3 | 142 | 0.71 |
|  | 7 | 1.3 | 136 | 0.56 |
|  | 8 | 1.3 | 130 | 0.47 |
|  | 10 | 1.3 | 120 | 0.35 |
|  | 12 | 1.3 | 111 | 0.28 |
|  | 20 | 1.3 | 86 | 0.15 |
|  | 50 | 1.3 | 47 | 0.06 |
| 2.0 lpm constant flow rate | 4.5 | 2.0 | 236 | 1.81 |
|  | 5 | 2.0 | 230 | 1.48 |
|  | 6 | 2.0 | 219 | 1.09 |
|  | 8 | 2.0 | 200 | 0.72 |
|  | 12 | 2.0 | 171 | 0.42 |
|  | 20 | 2.0 | 132 | 0.23 |
|  | 50 | 2.0 | 72 | 0.09 |
| 1.0 m/s constant air velocity | 5.0 | 1.4 | 155 | 1.00 |
|  | 8 | 2.8 | 279 | 1.00 |
|  | 20 | 8.6 | 566 | 1.00 |

Five repetition runs were carried out for each tube size and flow rate combination. Between adjacent runs there were at least 5 minutes wait time for the Spraytec system to be purged. In each run, real time particle size distributions were measured in the Spraytec laser diffraction system at a sampling rate of 2500 per second, the volume distribution median (Dv50) was averaged over a puff duration of 4 seconds. Measurement results were averaged and the standard deviations were calculated to indicate errors as shown in section 4 below.

2.2. Turbulence tube testing

The Reynolds numbers in Table 1 are all well below 1000, therefore, it is considered fair to assume all the experiments in section 2.1 would be under conditions of laminar flow. Further experiments were carried out and reported in this section to investigate the role of turbulence.

Turbulence intensity was introduced as a quantitative parameter to assess the level of turbulence. The definition and simulation of turbulence intensity is discussed below (see section 3.2).

Different device designs were considered in order to introduce turbulence. In the experiments reported here, jetting panels were added in the existing 12mm rectangular tubes upstream of the wick. This approach enables direct comparison between different devices as they all have highly similar geometry, with turbulence intensity being the only variable.

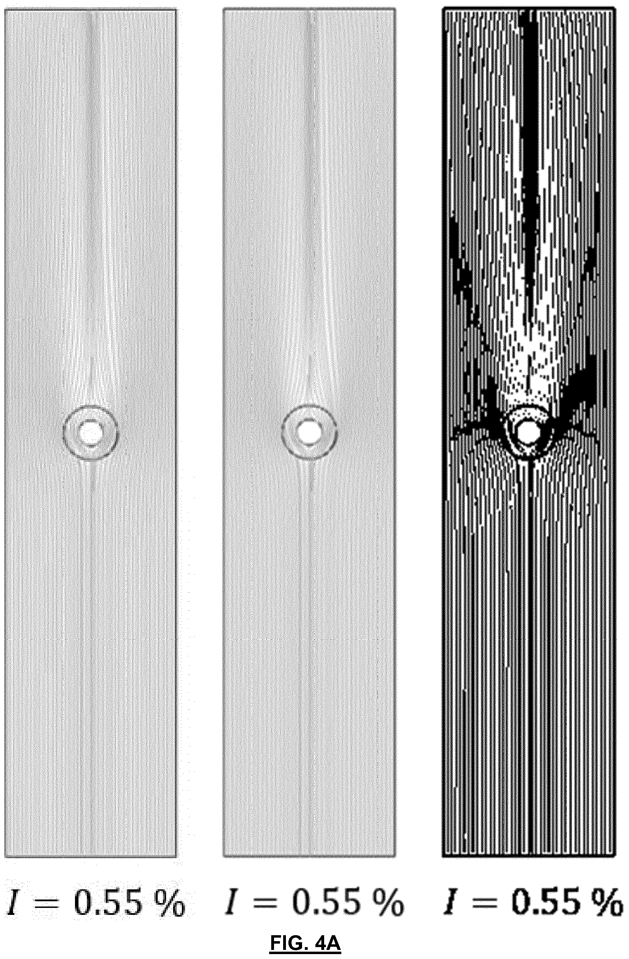

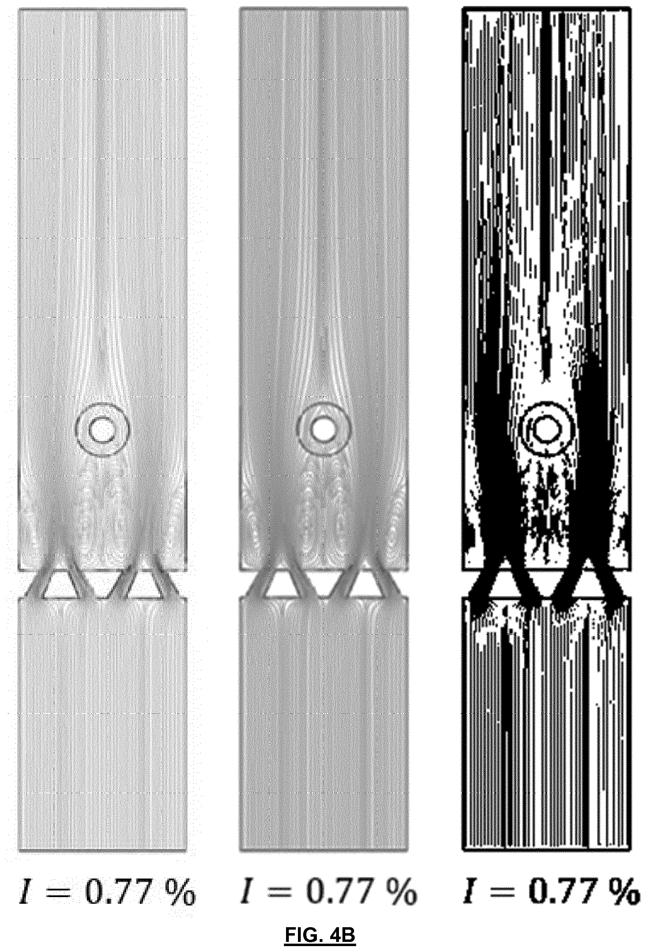

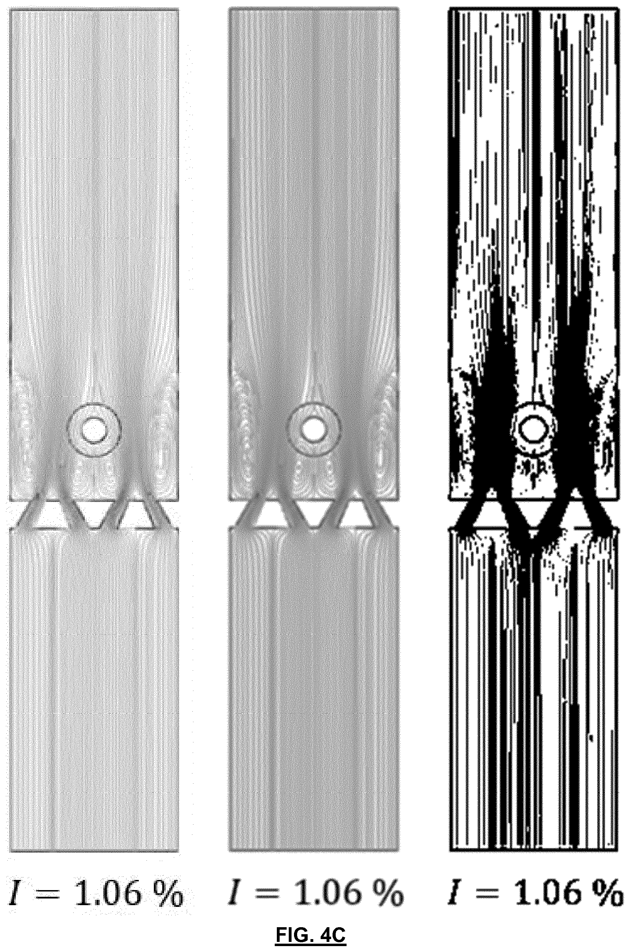

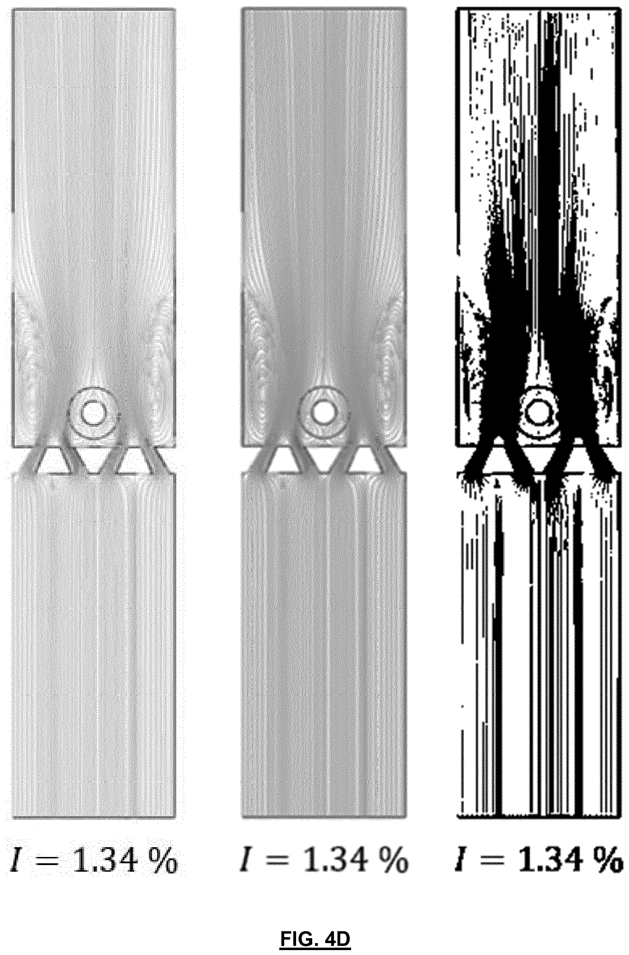

Figures 4A-4D show air flow streamlines in the four devices used in this turbulence study. Figure 4A is a standard 12mm rectangular tube with wick and coil installed as explained in the previous section, with no jetting panel. Figure 4B has a jetting panel located 10mm below (upstream from) the wick. Figure 4C has the same jetting panel 5mm below the wick. Figure 4D has the same jetting panel 2.5mm below the wick. As can be seen from Figures 4B-4D, the jetting panel has an arrangement of apertures shaped and directed in order to promote jetting from the downstream face of the panel and therefore to promote turbulent flow. Accordingly, the jetting panel can introduce turbulence downstream, and the panel causes higher level of turbulence near the wick when it is positioned closer to the wick. As shown in Figures 4A-4D, the four geometries gave turbulence intensities of 0.55%, 0.77%, 1.06% and 1.34%, respectively, with Figure 4A being the least turbulent, and Figure 4D being the most turbulent.

For each of Figures 4A-4D, there are shown three modelling images. The image on the left shows the original image (colour in the original), the central image shows a greyscale version of the image and the right hand image shows a black and white version of the image. As will be appreciated, each version of the image highlights slightly different features of the flow. Together, they give a reasonable picture of the flow conditions at the wick.

These four devices were operated to generate aerosols following the procedure explained above (section 2.1) using a flow rate of 1.3 lpm and the generated aerosols were tested for particle size in the Spraytec laser diffraction system.

2.3. High temperature testing

This experiment aimed to investigate the influence of inflow air temperature on aerosol particle size, in order to investigate the effect of vapour cooling rate on aerosol generation.

The experimental set up is shown in Figure 5. The testing used a Carbolite Gero EHA 12300B tube furnace 3210 with a quartz tube 3220 to heat up the air. Hot air in the tube furnace was then led into a transparent housing 3158 that contains the EVP device 3150 to be tested. A thermocouple meter 3410 was used to assess the temperature of the air pulled into the EVP device. Once the EVP device was activated, the aerosol was pulled into the Spraytec laser diffraction system 3310 via a silicone connector 3320 for particle size measurement.

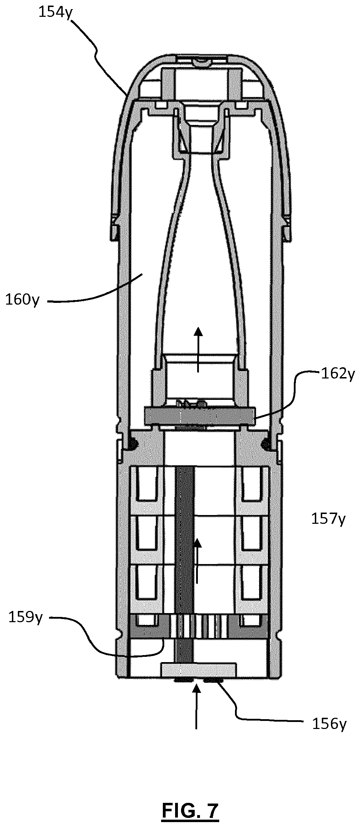

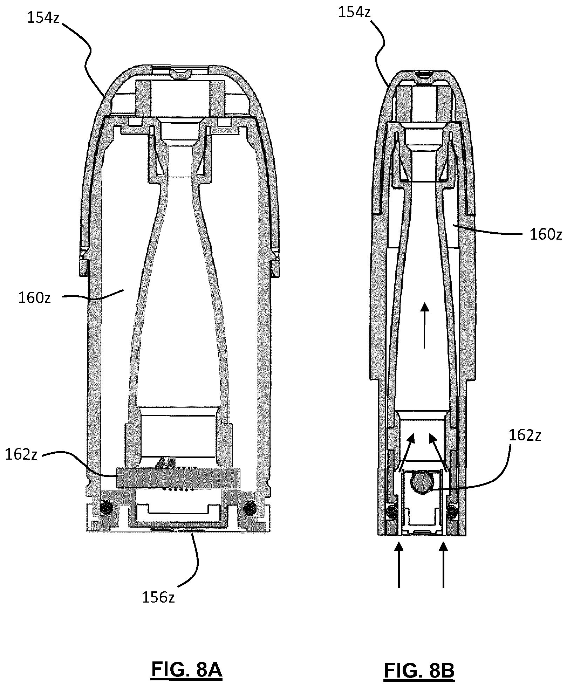

Three smoking substitute apparatuses (referred to as "pods") were tested in the study: pod 1 is the commercially available "myblu optimised" pod (Figure 6); pod 2 is a pod featuring an extended inflow path upstream of the wick (Figure 7); and pod 3 is pod with the wick located in a stagnant vaporisation chamber and the inlet air bypassing the vaporisation chamber but entraining the vapour from an outlet of the vaporisation chamber (Figures 8A and 8B).

Pod 1, shown in longitudinal cross sectional view (in the width plane) in Figure 6, has a main housing that defines a tank 160x holding an e-liquid aerosol precursor. Mouthpiece 154x is formed at the upper part of the pod. Electrical contacts 156x are formed at the lower end of the pod. Wick 162x is held in a vaporisation chamber. The air flow direction is shown using arrows.

Pod 2, shown in longitudinal cross sectional view (in the width plane) in Figure 7, has a main housing that defines a tank 160y holding an e-liquid aerosol precursor. Mouthpiece 154y is formed at the upper part of the pod. Electrical contacts 156y are formed at the lower end of the pod. Wick 162y is held in a vaporisation chamber. The air flow direction is shown using arrows. Pod 2 has an extended inflow path (plenum chamber 157y) with a flow conditioning element 159y, configured to promote reduced turbulence at the wick 162y.

Figure 8A shows a schematic longitudinal cross sectional view of pod 3. Figure 8B shows a schematic longitudinal cross sectional view of the same pod 3 in a direction orthogonal to the view taken in Figure 8A. Pod 3 has a main housing that defines a tank 160z holding an e-liquid aerosol precursor. Mouthpiece 154z is formed at the upper part of the pod. Electrical contacts 156z are formed at the lower end of the pod. Wick 162z is held in a vaporisation chamber. The air flow direction is shown using arrows. Pod 3 uses a stagnant vaporiser chamber, with the air inlets bypassing the wick and picking up the vapour/aerosol downstream of the wick.

All three pods were filled with the same e-liquid (1.6% freebase nicotine, 65:35 PG/VG ratio, no added flavour). Three experiments were carried out for each pod: 1) standard measurement in ambient temperature; 2) only the inlet air was heated to 50 °C; and 3) both the inlet air and the pods were heated to 50 °C. Five repetition runs were carried out for each experiment and the Dv50 results were taken and averaged.

### 3. Modelling work

In this study, modelling work was performed using COMSOL Multiphysics 5.4, engaged physics include: 1) laminar single-phase flow; 2) turbulent single-phase flow; 3) laminar two-phase flow; 4) heat transfer in fluids; and (5) particle tracing. Data analysis and data visualisation were mostly completed in MATLAB R2019a.

### 3.1. Velocity modelling

Air velocity in the vicinity of the wick is believed to play an important role in affecting particle size. In section 2.1, the air velocity was calculated by dividing the flow rate by the intersection area, which is referred to as "calculated velocity" in this work. This involves a very crude simplification that assumes velocity distribution to be homogeneous across the intersection area.

In order to increase reliability of the work, computational fluid dynamics (CFD) modelling was performed to obtain more accurate velocity values:

1) The average velocity in the vicinity of the wick (defined as a volume from the wick surface to 1mm away from the wick surface)

2) The maximum velocity in the vicinity of the wick (defined as a volume from the wick surface to 1mm away from the wick surface)

*Table 2. Average and maximum velocity in the vicinity of wick surface obtained from CFD modelling*

|  | Tube size [mm] | Flow rate [lpm] | Calculated velocity* [m/s] | Average velocity** [m/s] | Maximum Velocity** [m/s] |
|---|---|---|---|---|---|
| 1.3 lpm constant flow rate | 4.5 | 1.3 | 1.17 | 0.99 | 1.80 |
|  | 6 | 1.3 | 0.71 | 0.66 | 1.22 |
|  | 7 | 1.3 | 0.56 | 0.54 | 1.01 |
|  | 8 | 1.3 | 0.47 | 0.46 | 0.86 |
|  | 10 | 1.3 | 0.35 | 0.35 | 0.66 |
|  | 12 | 1.3 | 0.28 | 0.27 | 0.54 |
|  | 20 | 1.3 | 0.15 | 0.15 | 0.32 |
|  | 50 | 1.3 | 0.06 | 0.05 | 0.12 |
| 2.0 lpm constant flow rate | 4.5 | 2.0 | 1.81 | 1.52 | 2.73 |
|  | 5 | 2.0 | 1.48 | 1.31 | 2.39 |
|  | 6 | 2.0 | 1.09 | 1.02 | 1.87 |
|  | 8 | 2.0 | 0.72 | 0.71 | 1.31 |
|  | 12 | 2.0 | 0.42 | 0.44 | 0.83 |
|  | 20 | 2.0 | 0.23 | 0.24 | 0.49 |
|  | 50 | 2.0 | 0.09 | 0.08 | 0.19 |
| * Calculated by dividing flow rate with intersection area | | | | | |
| ** Obtained from CFD modelling | | | | | |

The CFD model uses a laminar single-phase flow setup. For each experiment, the outlet was configured to a corresponding flowrate, the inlet was configured to be pressure-controlled, the wall conditions were set as "no slip". A 1 mm wide ring-shaped domain (wick vicinity) was created around the wick surface, and domain probes were implemented to assess the average and maximum magnitudes of velocity in this ring-shaped wick vicinity domain.

The CFD model outputs the average velocity and maximum velocity in the vicinity of the wick for each set of experiments carried out in section 2.1. The outcomes are reported in Table 2.

3.2. Turbulence modelling

Turbulence intensity (I) is a quantitative value that represents the level of turbulence in a fluid flow system. It is defined as the ratio between the root-mean-square of velocity fluctuations, $u'$, and the Reynolds-averaged mean flow velocity, $U$:

$$I = \frac{u'}{U} = \frac{\sqrt{\frac{1}{3}\left(u'^2_x + u'^2_y + u'^2_z\right)}}{\sqrt{\overline{u_x}^2 + \overline{u_y}^2 + \overline{u_z}^2}} = \frac{\sqrt{\frac{1}{3}\left[\left(u_x - \overline{u_x}\right)^2 + \left(u_y - \overline{u_y}\right)^2 + +\left(u_z - \overline{u_z}\right)^2\right]}}{\sqrt{\overline{u_x}^2 + \overline{u_y}^2 + \overline{u_z}^2}}$$

where $u_x$, $u_y$ and $u_z$ are the x-, y- and z-components of the velocity vector, $\overline{u_x}$, $\overline{u_y}$, and $\overline{u_z}$ represent the average velocities along three directions.

Higher turbulence intensity values represent higher levels of turbulence. As a rule of thumb, turbulence intensity below 1% represents a low-turbulence case, turbulence intensity between 1% and 5% represents a medium-turbulence case, and turbulence intensity above 5% represents a high-turbulence case.

In this study, turbulence intensity was obtained from CFD simulation using turbulent single-phase setup in COMSOL Multiphysics. For each of the four experiments explained in section 2.2, the outlet was set to 1.3 lpm, the inlet was set to be pressure-controlled, and all wall conditions were set to be "no slip".

Turbulence intensity was assessed within the volume up to 1 mm away from the wick surface (defined as the wick vicinity domain). For the four experiments explained in section 2.2, the turbulence intensities are 0.55%, 0.77%, 1.06% and 1.34%, respectively, as also shown in Figures 4A-4D.

3.3. Cooling rate modelling

The cooling rate modelling involves three coupling models in COMSOL Multiphysics: 1) laminar two-phase flow; 2) heat transfer in fluids, and 3) particle tracing. The model is setup in three steps:

1) Set up two phase flow model

Laminar mixture flow physics was selected in this study. The outlet was configured in the same way as in section 3.1. However, this model includes two fluid phases released from two separate inlets: the first one is the vapour released from wick surface, at an initial velocity of 2.84 cm/s (calculated based on 5 mg total particulate mass over 3 seconds puff duration) with initial velocity direction normal to the wick surface; the second inlet is air influx from the base of tube, the rate of which is pressure-controlled.

2) Set up two-way coupling with heat transfer physics

The inflow and outflow settings in heat transfer physics was configured in the same way as in the two-phase flow model. The air inflow was set to 25 °C, and the vapour inflow was set to 209 °C (boiling temperature of the e-liquid formulation). In the end, the heat transfer physics is configured to be two-way coupled with the laminar mixture flow physics. The above model reaches steady state after approximately 0.2 second with a step size of 0.001 second.

3) Set up particle tracing

A wave of 2000 particles were release from wick surface at t = 0.3 second after the two-phase flow and heat transfer model has stabilised. The particle tracing physics has one-way coupling with the previous model, which means the fluid flow exerts dragging force on the particles, whereas the particles do not exert counterforce on the fluid flow. Therefore, the particles function as moving probes to output vapour temperature at each timestep.

The model outputs average vapour temperature at each time steps. A MATLAB script was then created to find the time step when the vapour cools to a target temperature (50°C or 75°C), based on which the vapour cooling rates were obtained (Table 3).

*Table 3. Average vapour cooling rate obtained from Multiphysics modelling*

| | Tube size [mm] | Flow rate [lpm] | Cooling rate to 50°C [°C/ms] | Cooling rate to 75°C [°C/ms] |
|---|---|---|---|---|
| 1.3 lpm constant flow rate | 4.5 | 1.3 | 11.4 | 44.7 |
| | 6 | 1.3 | 5.48 | 14.9 |
| | 7 | 1.3 | 3.46 | 7.88 |
| | 8 | 1.3 | 2.24 | 5.15 |
| | 10 | 1.3 | 1.31 | 2.85 |
| | 12 | 1.3 | 0.841 | 1.81 |
| | 20 | 1.3 | 0* | 0.536 |
| | 50 | 1.3 | 0 | 0 |
| 2.0 lpm constant flow rate | 4.5 | 2.0 | 19.9 | 670 |
| | 5 | 2.0 | 13.3 | 67 |
| | 6 | 2.0 | 8.83 | 26.8 |
| | 8 | 2.0 | 3.61 | 8.93 |
| | 12 | 2.0 | 1.45 | 3.19 |
| | 20 | 2.0 | 0.395 | 0.761 |
| | 50 | 2.0 | 0 | 0 |
| * Zero cooling rate when the average vapour temperature is still above target temperature after 0.5 second | | | | |

4. <u>Results and discussions</u>

Particle size measurement results for the rectangular tube testing are shown in Table 4. For every tube size and flow rate combination, five repetition runs were carried out in the Spraytec laser diffraction system. The Dv50 values from five repetition runs were averaged, and the standard deviations were calculated to indicate errors, as shown in Table 4.

In this section, the roles of different factors affecting aerosol particle size will be discussed based on experimental and modelling results.

*Table 4. Particle size measurement results for the rectangular tube testing*

| | Tube size [mm] | Flow rate [lpm] | Dv50 average [μm] | Dv50 standard deviation [μm] |
|---|---|---|---|---|
| 1.3 lpm constant flow rate | 4.5 | 1.3 | 0.971 | 0.125 |
| | 6 | 1.3 | 1.697 | 0.341 |
| | 7 | 1.3 | 2.570 | 0.237 |
| | 8 | 1.3 | 2.705 | 0.207 |
| | 10 | 1.3 | 2.783 | 0.184 |
| | 12 | 1.3 | 3.051 | 0.325 |
| | 20 | 1.3 | 3.116 | 0.354 |
| | 50 | 1.3 | 3.161 | 0.157 |

(continued)

|  | Tube size [mm] | Flow rate [lpm] | Dv50 average [μm] | Dv50 standard deviation [μm] |
|---|---|---|---|---|
| 2.0 lpm constant flow rate | 4.5 | 2.0 | 0.568 | 0.039 |
|  | 5 | 2.0 | 0.967 | 0.315 |
|  | 6 | 2.0 | 1.541 | 0.272 |
|  | 8 | 2.0 | 1.646 | 0.363 |
|  | 12 | 2.0 | 3.062 | 0.153 |
|  | 20 | 2.0 | 3.566 | 0.260 |
|  | 50 | 2.0 | 3.082 | 0.440 |
| 1.0 m/s constant air velocity | 5.0 | 1.4 | 1.302 | 0.187 |
|  | 8 | 2.8 | 1.303 | 0.468 |
|  | 20 | 8.6 | 1.463 | 0.413 |

4.1. Decouple the factors affecting particle size

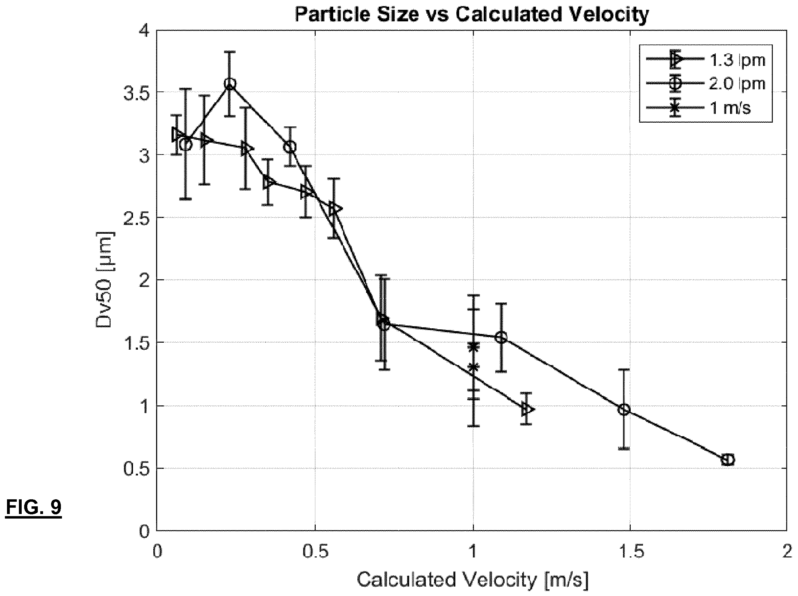

The particle size (Dv50) experimental results are plotted against calculated air velocity in Figure 9. The graph shows a strong correlation between particle size and air velocity.

Different size tubes were tested at two flow rates: 1.3 lpm and 2.0 lpm. Both groups of data show the same trend that slower air velocity leads to larger particle size. The conclusion was made more convincing by the fact that these two groups of data overlap well in Figure 9: for example, the 6mm tube delivered an average Dv50 of 1.697 μm when tested at 1.3 lpm flow rate, and the 8mm tube delivered a highly similar average Dv50 of 1.646 μm when tested at 2.0 lpm flow rate, as they have similar air velocity of 0.71 and 0.72 m/s, respectively.

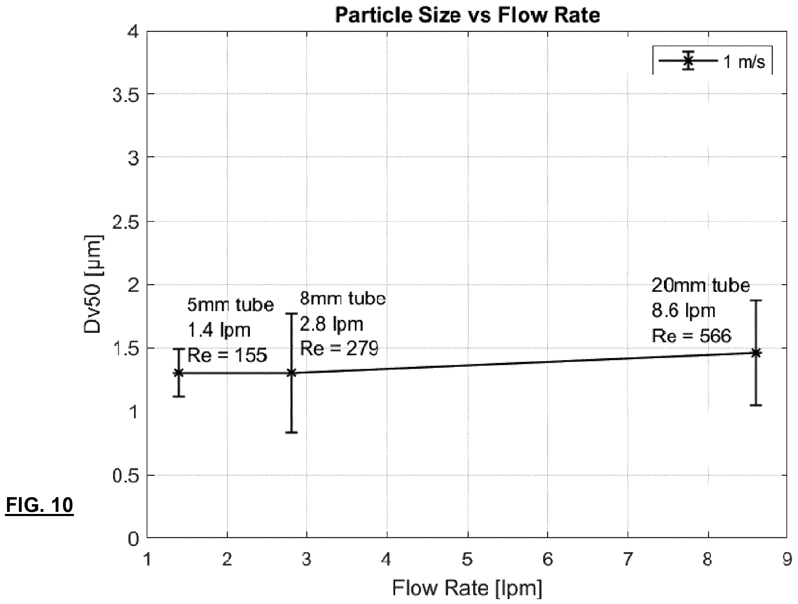

In addition, Figure 10 shows the results of three experiments with highly different setup arrangements: 1) 5mm tube measured at 1.4 lpm flow rate with Reynolds number of 155; 2) 8mm tube measured at 2.8 lpm flow rate with Reynolds number of 279; and 3) 20mm tube measured at 8.6 lpm flow rate with Reynolds number of 566. It is relevant that these setup arrangements have one similarity: the air velocities are all calculated to be 1 m/s. Figure 10 shows that, although these three sets of experiments have different tube sizes, flow rates and Reynolds numbers, they all delivered similar particle sizes, as the air velocity was kept constant. These three data points were also plotted out in Figure 9 (1 m/s data with star marks) and they tie in nicely into particle size-air velocity trendline.

The above results lead to a strong conclusion that air velocity is an important factor affecting the particle size of EVP devices. Relatively large particles are generated when the air travels with slower velocity around the wick. It can also be concluded that flow rate, tube size and Reynolds number are not necessarily independently relevant to particle size, providing the air velocity is controlled in the vicinity of the wick.

4.2. Further consideration of velocity

In Figure 9 the "calculated velocity" was obtained by dividing the flow rate by the intersection area, which is a crude simplification that assumes a uniform velocity field. In order to increase reliability of the work, CFD modelling has been performed to assess the average and maximum velocities in the vicinity of the wick. In this study, the "vicinity" was defined as a volume from the wick surface up to 1 mm away from the wick surface.

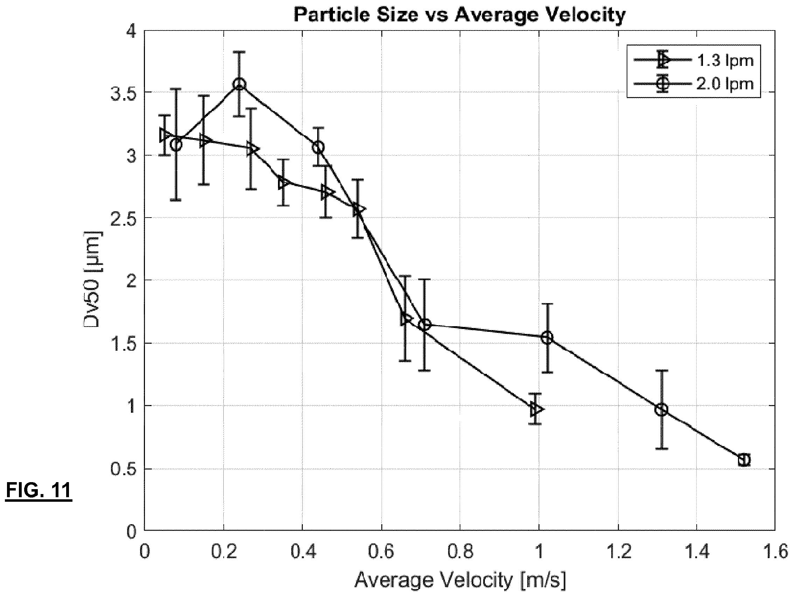

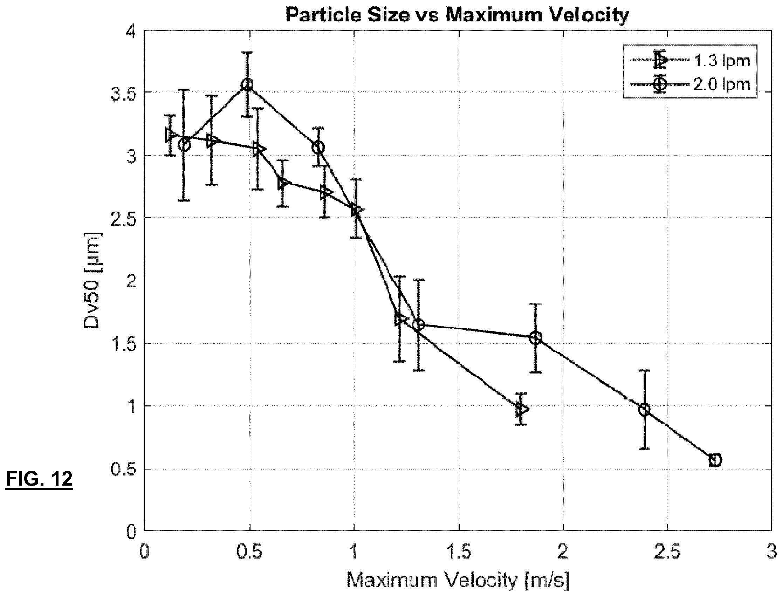

The particle size measurement data were plotted against the average velocity (Figure 11) and maximum velocity (Figure 12) in the vicinity of the wick, as obtained from CFD modelling.

The data in these two graphs indicates that in order to obtain an aerosol with Dv50 larger than 1 μm, the average velocity should be less than or equal to 1.2 m/s in the vicinity of the wick and the maximum velocity should be less than or equal to 2.0 m/s in the vicinity of the wick.

Furthermore, in order to obtain an aerosol with Dv50 of 2 μm or larger, the average velocity should be less than or equal to 0.6 m/s in the vicinity of the wick and the maximum velocity should be less than or equal to 1.2 m/s in the vicinity of the wick.

It is considered that typical commercial EVP devices deliver aerosols with Dv50 around 0.5 μm, and there is no commercially available device that can deliver aerosol with Dv50 exceeding 1 μm. It is considered that typical commercial EVP devices have average velocity of 1.5-2.0 m/s in the vicinity of the wick.

### 4.3. The role of turbulence

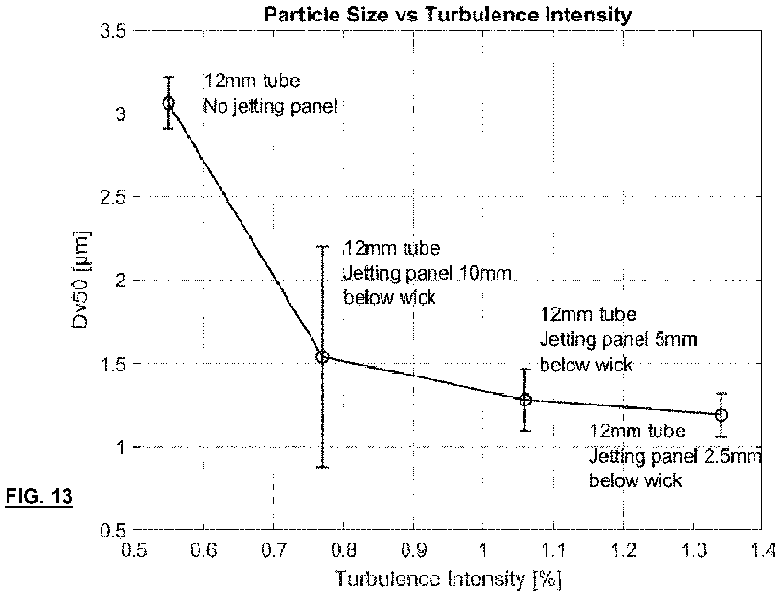

The role of turbulence has been investigated in terms of turbulence intensity, which is a quantitative characteristic that indicates the level of turbulence. In this work, four tubes of different turbulence intensities were used to general aerosols which were measured in the Spraytec laser diffraction system. The particle size (Dv50) experimental results are plotted against turbulence intensity in Figure 13.

The graph suggests a correlation between particle size and turbulence intensity, that lower turbulence intensity is beneficial for obtaining larger particle size. It is noted that when turbulence intensity is above 1% (medium-turbulence case), there are relatively large measurement fluctuations. In Figure 13, the tube with a jetting panel 10mm below the wick has the largest error bar, because air jets become unpredictable near the wick after traveling through a long distance.

The results clearly indicate that laminar air flow is favourable for the generation of aerosols with larger particles, and that the generation of large particle sizes is jeopardised by introducing turbulence. In Figure 13, the 12mm standard rectangular tube (without jetting panel) delivers above 3 $\mu$m particle size (Dv50). The particle size values reduced by at least a half when jetting panels were added to introduce turbulence.

### 4.4. Vapour cooling rate

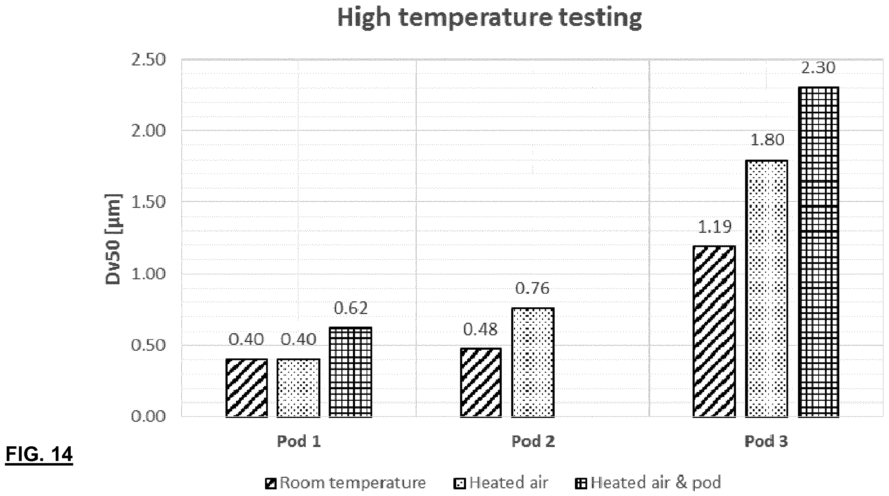

Figure 14 shows the high temperature testing results. Larger particle sizes were observed from all 3 pods when the temperature of inlet air increased from room temperature (23°C) to 50 °C. When the pods were heated as well, two of the three pods saw even larger particle size measurement results, while pod 2 was unable to be measured due to significant amount of leakage.

Without wishing to be bound by theory, the results are in line with the inventors' insight that control over the vapour cooling rate provides an important degree of control over the particle size of the aerosol. As reported above, the use of a slow air velocity can have the result of the formation of an aerosol with large Dv50. It is considered that this is due to slower air velocity allowing a slower cooling rate of the vapour.

Another conclusion related to laminar flow can also be explained by a cooling rate theory: laminar flow allows slow and gradual mixing between cold air and hot vapour, which means the vapour can cool down in slower rate when the airflow is laminar, resulting in larger particle size.

The results in Figure 14 further validate this cooling rate theory: when the inlet air has higher temperature, the temperature difference between hot vapour and cold air becomes smaller, which allows the vapour to cool down at a slower rate, resulting in larger particle size; when the pods were heated as well, this mechanism was exaggerated even more, leading to an even slower cooling rate and an even larger particle size.

### 4.5. Further consideration of vapour cooling rate

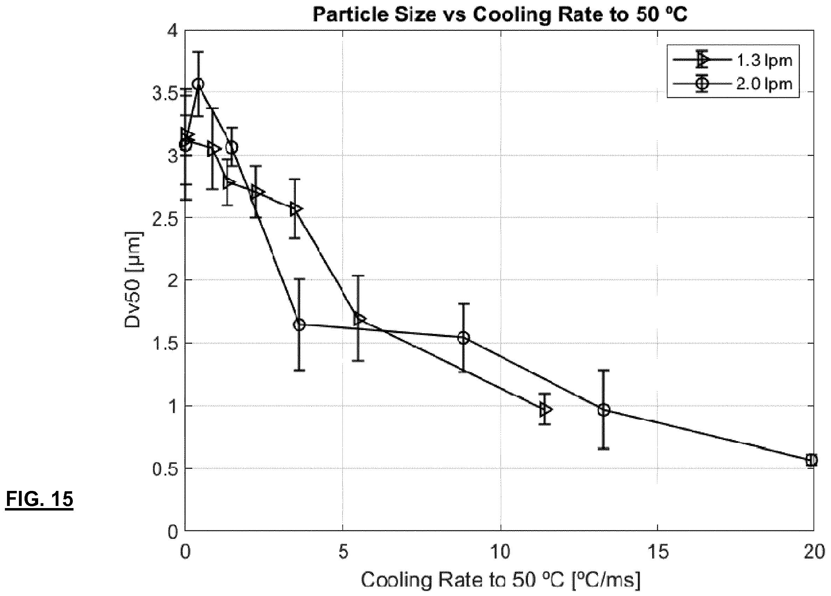

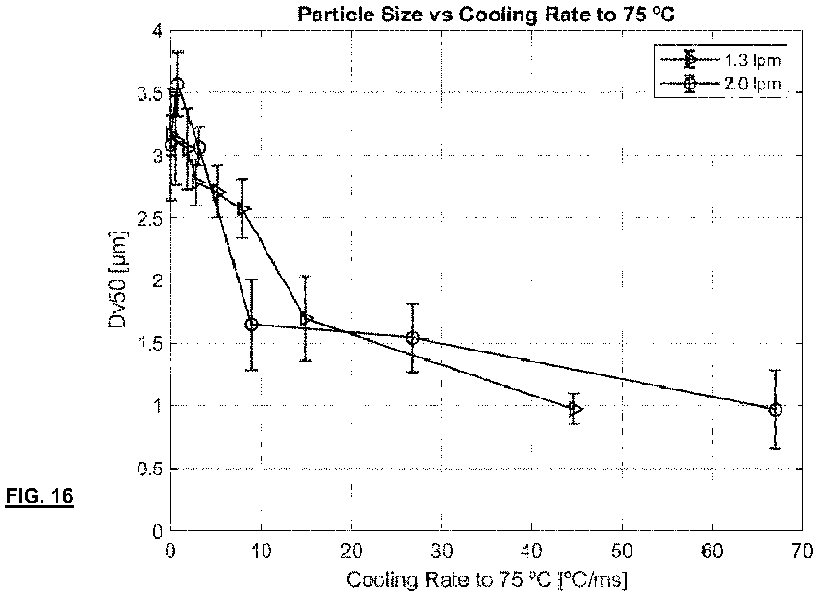

In section 3.3, the vapour cooling rates for each tube size and flow rate combination were obtained via multiphysics simulation. In Figure 15 and Figure 16, the particle size measurement results were plotted against vapour cooling rate to 50°C and 75°C, respectively.

The data in these graphs indicates that in order to obtain an aerosol with Dv50 larger than 1 $\mu$m, the apparatus should be operable to require more than 16 ms for the vapour to cool to 50°C, or an equivalent (simplified to an assumed linear) cooling rate being slower than 10 °C/ms. From an alternative viewpoint, in order to obtain an aerosol with Dv50 larger than 1 $\mu$m, the apparatus should be operable to require more than 4.5 ms for the vapour to cool to 75°C, or an equivalent (simplified to an assumed linear) cooling rate slower than 30 °C/ms.

Furthermore, in order to obtain an aerosol with Dv50 of 2 $\mu$m or larger, the apparatus should be operable to require more than 32 ms for the vapour to cool to 50°C, or an equivalent (simplified to an assumed linear) cooling rate being slower than 5 °C/ms. From an alternative viewpoint, in order to obtain an aerosol with Dv50 of 2 $\mu$m or larger, the apparatus should be operable to require more than 13 ms for the vapour to cool to 75°C, or an equivalent (simplified to an assumed linear) cooling rate slower than 10 °C/ms.

### 5. Conclusions of particle size experimental work

In this work, particle size (Dv50) of aerosols generated in a set of rectangular tubes was studied in order to decouple different factors (flow rate, air velocity, Reynolds number, tube size) affecting aerosol particle size. It is considered that air velocity is an important factor affecting particle size - slower air velocity leads to larger particle size. When air velocity was kept constant, the other factors (flow rate, Reynolds number, tube size) has low influence on particle size.

The role of turbulence was also investigated. It is considered that laminar air flow favours generation of large particles, and introducing turbulence deteriorates (reduces) the particle size.

Modelling methods were used to simulate the average air velocity, the maximum air velocity, and the turbulence intensity in the vicinity of the wick. A COMSOL model with three coupled physics has also been developed to obtain the vapour cooling rate.

All experimental and modelling results support a cooling rate theory that slower vapour cooling rate is a significant factor in ensuring larger particle size. Slower air velocity, laminar air flow and higher inlet air temperature lead to larger particle size, because they all allow vapour to cool down at slower rates.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

*List of features*

| | |
|---|---|
| 110 | E-cigarette system |
| 120 | Main body |
| 150 | E-cigarette consumable |
| 152 | Tank |
| 154 | Mouthpiece |
| 156 | Electrical contacts |
| 157 | Slots |
| 158 | Window |
| 160 | E-liquid |
| 162 | Porous wick |
| 164 | Heating filament |
| 170 | Passage |
| 172 | Vaporisation chamber inlet |
| 174 | Outlet |
| 176 | Device air inlets |
| 178 | Inlet flow channel |
| 401 | Flow of e-liquid |
| 402 | Airflow from inlet |
| 403 | Vapour-containing airflow |
| 501 | Housing |

| 502a/b | Secondary air inlets |
| 504a/b | Shared airflow path |
| 506a/b | Bypass air inlet |
| 508a/b | Bypass air duct |
| 510a/b | Adjustable airflow restrictor in bypass channel |
| 512a/b | Bypass air duct outlet |
| 514 | Plenum |
| 516 | Flow straightener |
| 518 | Coil and wick arrangement |
| 601a/b | Adjustable airflow restrictor in primary airflow path |
| 701a/b | Adjustable airflow restrictor in shared airflow path |

**Claims**

1. A smoking substitute apparatus comprising:

   a vaporiser chamber, including a vaporiser configured to vaporise a vaporisable liquid;
   a primary airflow path, which passes from a first air inlet of the smoking substitute apparatus through the vaporiser chamber, to an outlet of the smoking substitute apparatus;
   a secondary airflow path, which passes from a second air inlet of the smoking substitute apparatus to the outlet of the smoking substitute apparatus, said secondary airflow path bypassing the vaporiser chamber and passing through one or more bypass air ducts;
   wherein one or more adjustable airflow restrictors are disposed along either or both of the primary airflow path and the secondary airflow path, said adjustable airflow restrictors being adjustable by a user of the device to vary a draw resistance of the smoking substitute apparatus.

2. The smoking substitute apparatus of claim 1, wherein the or each airflow restrictor is a constriction along the respective airflow path, the constriction having an adjustable cross-section.

3. The smoking substitute apparatus of either claim 1 or claim 2, wherein the or each airflow restrictor is adjustable through the application of a force, by the user, to a portion of an outer housing of the smoking substitute apparatus.

4. The smoking substitute apparatus of either claim 1 or claim 2, wherein the or each airflow restrictor is adjustable through adjustment of a dial attached to a portion of an outer housing of the smoking substitute apparatus.

5. The smoking substitute apparatus of any preceding claim, wherein the or each airflow restrictor disposed along the secondary airflow path is located within the or each bypass air duct.

6. The smoking substitute apparatus of any preceding claim, wherein the or each airflow restrictor disposed along the primary airflow path is located between the first air inlet and a vaporiser chamber inlet of the vaporiser chamber.

7. The smoking substitute apparatus of any preceding claim, including two first air inlets, located on respectively opposite sides of the smoking substitute apparatus.

8. The smoking substitute apparatus of any preceding claim, including two second air inlets, located on respectively opposite sides of the smoking substitute apparatus.

9. The smoking substitute apparatus of any preceding claim, wherein the first air inlet provides air to both the primary and secondary airflow path.

10. The smoking substitute apparatus of any preceding claim, wherein the primary airflow path and secondary airflow path converge in the outlet of the smoking substitute apparatus.

11. The smoking substitute apparatus of any preceding claim, wherein the primary airflow path and the secondary airflow path partially overlap, and wherein the or each airflow restrictor is disposed along the overlapping portion of the airflow paths.

12. The smoking substitute apparatus of any preceding claim, the vaporiser chamber including a flow straightener located between a vaporiser chamber inlet and the vaporiser, configured to induce a laminar airflow over the vaporiser.

13. The smoking substitute apparatus of any preceding claim, the vaporiser chamber including a plenum, located between a vaporiser chamber inlet and the vaporiser, configured to reduce an airflow velocity over the vaporiser.

14. The smoking substitute apparatus of any preceding claim, including a vaporiser chamber outlet, located between the vaporiser and the outlet of the smoking substitute apparatus, wherein the vaporiser chamber outlet is a tapered chimney.

15. A smoking substitute system, including the smoking substitute apparatus of any preceding claim and a main body, the main body including a power source for the vaporiser.

**FIG. 1**

**FIG. 2**

1162    1164

1170

Constant depth
6 mm

Variable width
4.5mm - 50 mm

**FIG. 3**

$I = 0.55\ \%$    $I = 0.55\ \%$    $I = 0.55\ \%$

FIG. 4A

$$I = 0.77\,\%\qquad I = 0.77\,\%\qquad I = 0.77\,\%$$

FIG. 4B

$I = 1.06\,\%$  $I = 1.06\,\%$  $I = 1.06\,\%$

FIG. 4C

$$I = 1.34\,\% \qquad I = 1.34\,\% \qquad I = 1.34\,\%$$

**FIG. 4D**

**FIG. 5**

154x

160x

162x

156x

**FIG. 6**

**FIG. 7**

**FIG. 8A**         **FIG. 8B**

FIG. 9

EP 3 794 971 A1

**FIG. 10**

FIG. 11

EP 3 794 971 A1

**Particle Size vs Maximum Velocity**

FIG. 12

EP 3 794 971 A1

**Particle Size vs Turbulence Intensity**

FIG. 13

EP 3 794 971 A1

# High temperature testing

FIG. 14

EP 3 794 971 A1

Particle Size vs Cooling Rate to 50 °C

FIG. 15

EP 3 794 971 A1

FIG. 16

**FIG. 17**

**FIG. 18**

**FIG. 19**

**FIG. 20**

**FIG. 21**

**FIG. 22**

**FIG. 23**

Europäisches
Patentamt

European
Patent Office

Office européen
des brevets

# EUROPEAN SEARCH REPORT

**Application Number**

EP 19 19 8578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/040575 A1 (FONTEM HOLDINGS 1 BV [NL]; WENSLEY MARTIN [US] ET AL.) 17 March 2016 (2016-03-17) | 1-6,9-15 | INV. A24F40/10 A24F40/40 |
| Y | * page 4 - page 8; figures 1-4 * | 7,8 | A61M15/00 |
| Y | US 2017/325505 A1 (FORCE ERIC [CH] ET AL) 16 November 2017 (2017-11-16) * paragraph [0101] - paragraph [0106]; figures 1,2,10,11 * * paragraph [0037] - paragraph [0039] * | 7,8 | |
| A | US 2019/150520 A1 (FRASER RORY [GB] ET AL) 23 May 2019 (2019-05-23) * paragraph [0018] - paragraph [0076]; figures 1-4 * | 1-15 | |
| A | US 2018/360116 A1 (SCHMIDT RENE [DE] ET AL) 20 December 2018 (2018-12-20) * paragraphs [0031], [0062], [0109]; figures 2,3 * | 1-15 | |
| A | US 10 412 996 B2 (ALTRIA CLIENT SERVICES LLC [US]) 17 September 2019 (2019-09-17) * abstract; figure 1 * | 1-15 | TECHNICAL FIELDS SEARCHED (IPC) A24F A61M |
| A | WO 2004/050139 A2 (ALEXZA MOLECULAR DELIVERY CORP [US]) 17 June 2004 (2004-06-17) * figures 5A-5F,8A-8C * | 1-15 | |

The present search report has been drawn up for all claims

| Place of search | Date of completion of the search | Examiner |
|---|---|---|
| Munich | 11 March 2020 | Schwertfeger, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 8578

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2020

| Patent document cited in search report | Publication date | Patent family member(s) | Publication date |
|---|---|---|---|
| WO 2016040575 A1 | 17-03-2016 | CN 107847696 A<br>EP 3191162 A1<br>US 2017246405 A1<br>WO 2016040575 A1 | 27-03-2018<br>19-07-2017<br>31-08-2017<br>17-03-2016 |
| US 2017325505 A1 | 16-11-2017 | AR 103016 A1<br>CA 2962756 A1<br>CN 107889450 A<br>EP 3232841 A1<br>JP 2018500015 A<br>KR 20170095188 A<br>RU 2017124441 A<br>US 2017325505 A1<br>WO 2016096780 A1 | 12-04-2017<br>23-06-2016<br>06-04-2018<br>25-10-2017<br>11-01-2018<br>22-08-2017<br>17-01-2019<br>16-11-2017<br>23-06-2016 |
| US 2019150520 A1 | 23-05-2019 | AU 2017286499 A1<br>BR 112018075887 A2<br>CA 3026765 A1<br>CL 2018003426 A1<br>CN 109195654 A<br>EP 3468648 A1<br>JP 2019522963 A<br>KR 20190006540 A<br>PH 12018502452 A1<br>US 2019150520 A1<br>WO 2017216516 A1 | 13-12-2018<br>19-03-2019<br>21-12-2017<br>15-03-2019<br>11-01-2019<br>17-04-2019<br>22-08-2019<br>18-01-2019<br>21-10-2019<br>23-05-2019<br>21-12-2017 |
| US 2018360116 A1 | 20-12-2018 | CN 108926042 A<br>DE 102017111435 A1<br>EP 3407677 A1<br>JP 2018196374 A<br>KR 20180128853 A<br>US 2018360116 A1 | 04-12-2018<br>29-11-2018<br>28-11-2018<br>13-12-2018<br>04-12-2018<br>20-12-2018 |
| US 10412996 B2 | 17-09-2019 | US 2017172210 A1<br>US 2019373957 A1 | 22-06-2017<br>12-12-2019 |
| WO 2004050139 A2 | 17-06-2004 | AU 2003294231 A1<br>CA 2507265 A1<br>EP 1581281 A2<br>ES 2702606 T3<br>JP 2006507909 A<br>MX PA05005628 A<br>NZ 540209 A<br>US 2004099266 A1<br>WO 2004050139 A2 | 23-06-2004<br>17-06-2004<br>05-10-2005<br>04-03-2019<br>09-03-2006<br>16-08-2005<br>30-11-2006<br>27-05-2004<br>17-06-2004 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 19 19 8578

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2020

| Patent document cited in search report | Publication date | Patent family member(s) | Publication date |
|---|---|---|---|

EPO FORM P0459

For more details about this annex : see Official Journal of the European Patent Office, No. 12/82

page 2 of 2